# EUROPÄISCHE PATENTSCHRIFT

(11) **EP 2 475 335 B1**
(45) Veröffentlichungstag und Bekanntmachung des Hinweises auf die Patenterteilung: **17.02.2016**
(21) Anmeldenummer: 10755108.7
(22) Anmeldetag: 14.09.2010
(51) Int. Cl.: A61F 2/848, A61F 2/91, A61F 2/915

(54) **MEDIZINISCHES IMPLANTAT**
MEDICAL IMPLANT
IMPLANT MÉDICAL

(30) Priorität: 14.09.2009 DE 102009041025
(43) Veröffentlichungstag der Anmeldung: 18.07.2012
(73) Patentinhaber: Acandis GmbH & Co. KG, 76327 Pfinztal (DE)
(72) Erfinder: NAGL, Frank, 76137 Karlsruhe (DE); MAILÄNDER, Werner, 75331 Engelsbrand Grunbach (DE)
(74) Vertreter: Kilchert, Jochen
(86) Internationale Anmeldenummer: PCT/EP2010/005625
(87) Internationale Veröffentlichungsnummer: WO 2011/029631

(56) Entgegenhaltungen:
- WO-A1-99/65418
- WO-A1-2008/125145
- WO-A2-2007/058857
- DE-U1- 20 314 392
- US-A1- 2005 143 801
- US-A1- 2008 188 924

## Beschreibung

Die Erfindung betrifft ein medizinisches Implantat mit einer Wandung, die eine von einem komprimierten in einen expandierten Zustand überführbare und aus ersten Stegen gebildete Gitterstruktur umfasst, wobei die Gitterstruktur geschlossene Zellen mit jeweils einem Halteelement aufweist, das zur Verankerung der expandierten Gitterstruktur in einem Gefäß angepasst ist. Ein derartiges Implantat ist beispielsweise aus US 5,397,355 bekannt.

Im Bereich der interventionellen Neuroradiologie haben sich zwei grundsätzliche Stentdesigns zur Behandlung von Aneurysmen und Gefäßstenosen bewährt und zwar sogenannte Closed Cell Designs oder Open Cell Designs. In jüngere Zeit wurden verstärkt Closed Cell Designs eingesetzt, die den Vorteil haben, dass ein Stentsystem nach bereits erfolgter teilweiser Entlassung im Zielgebiet wieder in den Katheter eingezogen werden kann. Die auf dem Markt verfügbaren Stentsysteme, die in Kombination mit sogenannten Coils für die Therapie von Aneurysmen eingesetzt werden, können jedoch Schwächen in der Applikationssicherheit aufweisen. Nach dem Entlassen des Stentsystems wird ein Coilkatheter durch die Zellen des Stents in das Aneurysma eingeführt, um das Coil in das Aneurysma zu bringen. Dabei kann sich der Coilkatheter in den Spitzen der Zellen verhaken, so dass - bei unzureichender Verankerung des Stents im Blutgefäß - der Stent verrutschen kann. Eine unzureichende Verankerung des Stents im Blutgefäß kann beispielsweise durch eine zu geringe nach außen gerichtete Radialkraft oder durch die Ausprägung der Zellgeometrie bedingt sein. Zudem sind häufig Schwächen bei der Anpassung an die anatomischen Verhältnisse im neurocerebralen Gefäßsystem bei bekannten Stentsystemen zu beobachten.

Ein Stent mit einem Open Cell Design ist beispielsweise in US 2005/0143801 A1 beschrieben. Dabei weist der Stent Hakenelemente auf, die dazu dienen, eine Abdeckung auf dem Stent zu fixieren (Stent-Graft). Ein Zurückziehen des Stens in einen Katheter, beispielsweise um eine Fehlpositionierung zu korrigieren, ist nicht möglich, da sich die Hakenelemente beim Zurückziehen des Stents an der Katheterspitze verhaken.

Der Stent gemäß US 5,397,355 weist eine aus Stegen gebildete Gitterstruktur auf, die von einem komprimierten in einen expandierten Zustand überführt werden kann. Die Gitterstruktur ist aus geschlossenen Zellen gebildet (Closed Cell Design). Zur Verankerung der expandierten Gitterstruktur in einem Gefäß weisen die Zellen jeweils ein Halteelement auf, das im expandierten Zustand radial nach außen bewegt ist und über die Wandung des Stents hinausragt. Das Halteelement ist als Dorn mit einer scharfen Spitze ausgebildet, die sich im implantierten Zustand in die Gefäßwand bohrt. Dabei kann es zu Verletzungen kommen.

Eine weitere Möglichkeit, einen Stent im Gefäß zu verankern, ist in US 5,330,500 offenbart, die, ähnlich wie der vorstehend beschriebene bekannte Stent hakenförmige Spitzen in den Zellen aufweist, die sich im implantierten Zustand in die Gefäßwand bohren. US 5,800,526 beschreibt einen Stent mit einem Verankerungssystem, bei dem die Gitterstruktur so ausgebildet ist, dass diese abschnittsweise im implantierten Zustand über die Wandung des Stents vorsteht und den Stent gegen Dislokation fixiert. Bei dem Stent handelt es sich aber nicht um ein Closed Cell Design, sondern um ein Open Cell Design, das den Nachteil hat, dass dieses nur schlecht nach dem Entlassen in den Katheter zurückgezogen werden kann. Dasselbe gilt für den Stent gemäß US 2006/0100695 A1, der eine Gitterstruktur aufweist, die teilweise aus geschlossenen und teilweise aus offenen Zellen gebildet ist. Der Stent hat eine arzneimittelabgebende Funktion (drug eluting stent) und weist hierfür eine besonders große Oberfläche auf. Dazu sind verschiedene Möglichkeiten offenbart, unter anderem die Möglichkeit, die Stege eines offenzelligen Stentabschnittes durch Zwischenstege zu verbinden. Diese Zwischenstege haben nicht die Funktion eines Halteelements, sondern sollen ausschließlich die wirksame Stentoberfläche vergrößern.

WO 2008/125145 A1 offenbart einen Stent mit einer Gitterstruktur, die stellenweise unterbrochen ist. In den Unterbrechungen sind Halteelemente in Form von Fortsätzen oder V-förmig angeordneten Stegpaaren angeordnet. Diese Fortsätze bzw. Stegpaare sind jeweils mit Verbindern der Gitterstruktur gekoppelt. Die Verbinder bilden im Wesentlichen Gelenkstellen, so dass die Halteelemente unter Krafteinwirkung leicht ausweichen. Das birgt das Risiko, dass die Halteelemente im implantierten Zustand in den Blutfluss hineinragen und unerwünschte Verwirbelungen hervorrufen.

Der Erfindung liegt die Aufgabe zugrunde, ein medizinisches Implantat anzugeben, das eine Gitterstruktur mit geschlossenen Zellen aufweist und im Hinblick auf die Applikationssicherheit verbessert ist.

Erfindungsgemäß wird diese Aufgabe durch ein medizinisches Implantat mit den Merkmalen des Anspruchs 1 gelöst.

Die Erfindung beruht auf dem Gedanken, ein medizinisches Implantat mit einer Wandung anzugeben, die eine von einem komprimierten in einen expandierten Zustand überführbare und aus ersten Stegen gebildete Gitterstruktur umfasst, wobei die Gitterstruktur geschlossene Zellen mit jeweils einem Halteelement aufweist. Das Halteelement ist zur Verankerung der expandierten Gitterstruktur in einem Gefäß angepasst. Das Halteelement weist wenigstens zwei zweite Stege auf, die einerseits miteinander verbunden sind und eine Spitze bilden, die in die Zelle ragt. Andererseits sind die zweiten Stege des Halteelements mit den ersten Stegen der Zelle verbunden.

Die Erfindung hat mehrere Vorteile. Die Ausbildung des Halteelements mit wenigstens zwei Stegen bietet die Möglichkeit, eine atraumatische Ausgestaltung des Halteelements zu verwirklichen im Gegensatz zu dem Halteelement gemäß US 5,397,355. Überdies führt die stegförmige Ausbildung des Halteelements dazu, dass auf dem Umfang des Implantats die Anzahl der Stege und somit die auf die Gefäßwand wirkende Radialkraft erhöht wird. Dadurch ergibt sich der Vorteil, dass die Applikationssicherheit verbessert wird, insbesondere im Hinblick auf eine Vermeidung der Dislokation des Stents, ohne dass dabei der Hauptvorteil von Closed Cell Stentsystemen beeinträchtigt wird, nämlich die Möglichkeit, das Stentsystem nach erfolgter Teilentlassung im Zielgebiet wieder in den Katheter einzuziehen.

Bei einer bevorzugten Ausführungsform der Erfindung bilden die zweiten Stege des Halteelements mit den ersten Stegen einer Zelle der Gitterstruktur eine weitere Zelle, die in der Zelle der Gitterstruktur angeordnet ist. Neben der damit erreichbaren Radialkrafterhöhung führt die Anordnung der Zelle in der Zelle dazu, dass das Implantat, wenn dieses aufgrund einer Gefäßform in Biegung geht, die Spitze des Halteelements über die Wandung hinaus in die Gefäßwand hinein bewegt wird und das Implantat sicher verankert.

Die Form der weiteren Zelle kann der Form der Zelle der Gitterstruktur entsprechen. Dies bedeutet, dass die in die größere Zelle der Gitterstruktur einbeschriebene kleinere Zelle, die die Haltefunktion ausübt, die Form der größeren Zelle wiederholt. Dies hat zum einen fertigungstechnische Vorteile und zum anderen ist das Verformungsverhalten der weiteren Zelle besser vorhersehbar.

Die ersten Stege der Gitterstruktur sind erfindungsgemäß mit den zweiten Stegen des Halteelements jeweils zwischen zwei die Stege axial begrenzenden Verbindern verbunden. Insbesondere können die ersten Stege der Gitterstruktur mittig mit den zweiten Stegen des Halteelements verbunden sein. Dadurch wird eine gute Kraftübertragung von den ersten Stegen der Zelle auf die zweiten Stege des Halteelements erreicht.

Alternativ können die zweiten Stege des Halteelements mit jeweils einem axialen Ende der ersten Stege der Gitterstruktur verbunden sein, wobei das Halteelement einen die ersten Stege axial begrenzenden Verbinder ersetzt. Bei dieser alternativen, spannungsoptimierten Ausführungsform sind also die ersten Stege, insbesondere die axialen Enden der ersten Stege, mittelbar durch das Halteelement verbunden, das sich im Wesentlichen in die Zelle der Gitterstruktur hineinerstreckt. Das Halteelement kann ferner in einen Bereich außerhalb der Zelle der Gitterstruktur verlängert sein, so dass das Halteelement außerhalb der Zelle eine weitere Spitze bildet, die mit einer axial benachbarten Zelle durch einen Verbinder gekoppelt ist. Vorzugsweise ist das Halteelement bei dieser Ausführungsform symmetrisch ausgebildet, wobei die entsprechende Symmetrieachse auf dem Umfang der Gitterstruktur durch die axialen Enden der ersten Stege verläuft. Auf diese Weise wird die stressoptimierte bzw. spannungsoptimierte Ausgestaltung der Struktur verbessert.

Die Längsachse L' der weiteren Zelle kann mit einer Längsachse L" der zugehörigen Zelle der Gitterstruktur fluchten, wodurch die Auslenkung der Spitze des Halteelements zu einer guten Verankerung des Implantats führt.

Die Spitze des Halteelements kann zumindest auf der Höhe seitlicher Verbinder angeordnet sein, die in Umfangsrichtung nebeneinander angeordnete Zellen der Gitterstruktur verbinden. Dadurch wird die Möglichkeit geboten, dass die Spitze relativ weit über die Wandung ausgelenkt wird und somit entsprechend fest mit der Gefäßwand verankert wird. Die Spitze kann in distaler Richtung des Implantats weisen, wodurch die Möglichkeit verbessert wird, das teilweise in das Gefäß entlassene Implantat wieder in den Katheter einzuziehen. Bei einer besonders bevorzugten Ausführungsform sind wenigstens ein Randbereich, insbesondere zwei Randbereiche und wenigstens ein Zwischenbereich der Gitterstruktur in Längsrichtung der Wandung angeordnet, wobei die Halteelemente im Randbereich ausgebildet sind. Dieses Implantat eignet sich besonders gut zur Behandlung von Aneurysmen, da die Verankerungsfunktion auf die Randbereiche beschränkt werden kann und der Zwischenbereich die Stütz- und Verschlussfunktion übernehmen kann, um das Aneurysma und darin befindliche Coils gegen das Gefäßlumen abzuschließen. Die Erfindung ist nicht auf diesen Einsatzzweck eingeschränkt. Die Unterteilung des Implantats in Rand- und Zwischenbereiche ermöglicht eine Funktionstrennung, die auch für andere Einsätze vorteilhaft ist. Dabei übernehmen die Randbereiche oder zumindest ein Randbereich die Haltefunktion aufgrund der Halteelemente und der Zwischenbereich eine andere dem jeweiligen Therapiezweck entsprechende Funktion. Es ist auch möglich, die Halteelemente im Zwischenbereich vorzusehen und die Randbereiche für andere Funktionen auszubilden.

Bei einer weiteren bevorzugten Ausführungsform der Erfindung bilden die geschlossenen Zellen der Gitterstruktur mehrere in axialer Richtung der Wandung einander nachgeordnete Ringsegmente, wobei die Zellen eines Ringsegments wenigstens ein Halteelement, insbesondere alle Zellen des Ringsegments jeweils ein Halteelement aufweisen. Dadurch wird die Möglichkeit geschaffen, die Haltefunktion des Implantats auf einen bestimmten Bereich, nämlich auf ein oder mehrere Ringsegmente zu beschränken, und andere Implantatbereiche an andere Funktionen anzupassen.

Die Anzahl der distal angeordneten Ringsegmente mit Halteelementen kann größer sein als die Anzahl der proximal angeordneten Ringsegmente mit Halteelementen. Dadurch wird einerseits das Implantat beim Entlassen sicher im Gefäß fixiert, so dass eine genaue Positionierung des Implantats möglich ist. Andererseits wird dadurch die Möglichkeit verbessert, das Implantat in den Katheter wieder einzuziehen, da im proximalen Bereich eine geringere Anzahl von Ringsegmenten mit Halteelementen vorgesehen ist.

Es hat sich als besonders zweckmäßig herausgestellt, wenn die Zelle der Gitterstruktur eine rautenförmige Zelle umfasst. Die weitere Zelle, die in der Zelle der Gitterstruktur angeordnet ist, kann ebenfalls eine rautenförmige Zelle umfassen.

In einer bevorzugten Ausführungsform des Implantats ist vorgesehen, dass das Halteelement derart angepasst ist, dass sich die Spitze des Halteelements bei einer Krümmung der Gitterstruktur entlang einer Längsachse der Gitterstruktur selbsttätig radial nach außen aufstellt. Mit anderen Worten ist vorgesehen, dass das Halteelement in einem gekrümmten bzw. axial gebogenen Zustand der Gitterstruktur radial nach außen vorsteht und somit die Verankerung in einem Körpergefäß verbessern kann.

Vorzugsweise sind mehrere Halteelemente vorgesehen, die über den Umfang der Gitterstruktur verteilt angeordnet sind. Bei einer Krümmung der Gitterstruktur entlang einer Längsachse der Gitterstruktur können sich die Spitzen der Halteelemente, insbesondere nur der Halteelemente, die auf einer vom Krümmungsmittelpunkt abgewandten Seite der Gitterstruktur angeordnet sind, radial nach außen aufstellen. Bei einer axialen Krümmung der Gitterstruktur, beispielsweise bei Anordnung des Implantats in einer Gefäßkrümmung, kann ein einseitiges Aufstellen der Halteelemente erfolgen, wobei die Spitzen der Halteelemente radial nach außen gerichtet sind. Die Halteelemente stellen sich vorzugsweise auf der vom Krümmungsmittelpunkt abgewandten Seite der Gitterstruktur auf. Insbesondere stellen sich die Halteelemente auf der Seite der Gitterstruktur radial nach außen auf, die bei gekrümmter bzw. axial gebogener Anordnung der Gitterstruktur eine vergleichsweise höhere Streckung erfährt. Die Halteelemente, die auf einer gegenüberliegenden Seite der Gitterstruktur, also näher am Krümmungsmittelpunkt, angeordnet sind, erstrecken sich hingegen im Wesentlichen in der Wandungsebene bzw. werden nicht radial nach außen ausgelenkt. Im Allgemeinen ist vorgesehen, dass die radial nach außen gerichtete Auslenkung der Halteelemente bzw. der Spitzen der Halteelemente durch eine Krümmung der Gitterstruktur entlang einer Längsachse bzw. durch eine axiale Biegung der Gitterstruktur bewirkt wird.

In einer bevorzugten Ausgestaltung des erfindungsgemäßen Implantats sind mehrere Halteelemente vorgesehen, wobei die Spitzen aller Halteelemente in dieselbe Richtung, insbesondere in distale Richtung bezogen auf ein Zuführsystem, weisen. Durch die gleichgerichtete Anordnung der Halteelemente wird sichergestellt, dass das Implantat in ein Zuführsystem zurückziehbar ist, ohne dass die Spitzen der Halteelemente mit dem Zuführsystem oder der Gefäßwand verhaken. Daher ist besonders bevorzugt vorgesehen, dass die Spitzen aller Halteelemente bezogen auf das Zuführsystem in distale Richtung, also vom Anwender des Zuführsystems weg, weisen.

Die Erfindung wird nachfolgend anhand von Ausführungsbeispielen unter Bezug auf die beigefügten schematischen Zeichnungen mit weiteren Einzelheiten näher erläutert. In diesen zeigen:
- Fig. 1: in Draufsicht einen in einer Ebene aufgespannten Stent nach einem erfindungsgemäßen Ausführungsbeispiel;
- Fig. 2: einen Ausschnitt des Stents gemäß Fig. 1 im proximalen Randbereich;
- Fig. 3: einen Ausschnitt des Stents gemäß Fig. 1 im Zwischenbereich 22, wobei eine rautenförmige geschlossene Zelle dargestellt ist;
- Fig. 4: einen Ausschnitt des Stents gemäß Fig. 1 im distalen Randbereich;
- Fig. 5: einen Ausschnitt des Stents im proximalen oder distalen Randbereich bei teilweiser Komprimierung;
- Fig. 6a, 6b, 6c: Darstellungen des Stents gemäß Fig. 1 im Zwischenbereich im implantierten Zustand;
- Fig. 7a, 7b, 7c: Darstellungen des Stents gemäß Fig. 1 im distalen oder proximalen Randbereich im implantierten Zustand;
- Fig. 8a, 8b, 8c: Darstellungen des Stents gemäß Fig. 1 im distalen oder proximalen Randbereich im implantierten Zustand, wobei der Stent gekrümmt ist,
- Fig. 9a, 9b, 9c: Darstellungen eines Stents im distalen oder proximalen Randbereich im implantierten Zustand nach einem weiteren, erfindungsgemäßen Ausführungsbeispiel;
- Fig. 10: in Draufsicht einen in einer Ebene aufgespannten Stent nach einem weiteren, erfindungsgemäßen Ausführungsbeispiel;
- Fig. 11: in Draufsicht einen in einer Ebene aufgespannten Stent nach einem weiteren, erfindungsgemäßen Ausführungsbeispiel;
- Fig. 12a, 12b, 12c: jeweils einen Ausschnitt des Stents gemäß Fig. 11, wobei unterschiedliche Längenverhältnisse dargestellt sind;
- Fig. 13a, 13b: jeweils einen Längsschnitt durch ein Körperhohlorgan mit einem implantierten Stent gemäß Fig. 11;
- Fig. 14a, 14b: jeweils zwei Ansichten zweier Zellen eines Stents, wobei die Verankerung der Stege bzw. Halteelemente des Stents in der Gefäßwand eines Körperhohlorgans dargestellt ist;
- Fig. 15a: zwei Ansichten einer Zelle eines Stents mit einem Halteelement, wobei die Zelle in einem geradlinig verlaufenden Körperhohlorgan angeordnet ist;
- Fig. 15b: zwei Ansichten einer Zelle eines Stents mit einem Haltemittel, wobei die Zelle in einem gekrümmt verlaufenden Körperhohlorgan angeordnet ist; und

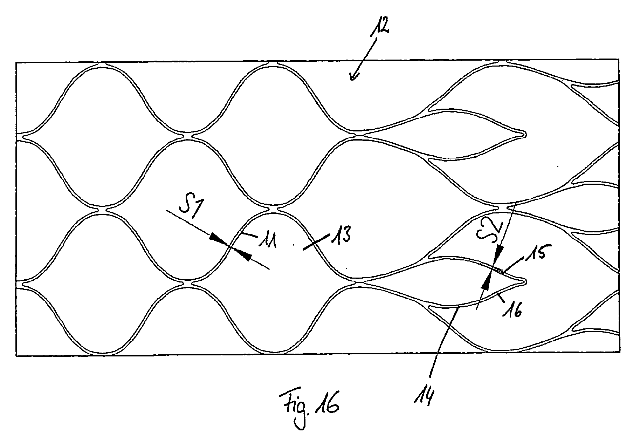

- Fig. 16: einen Ausschnitt des Stents gemäß Fig. 11, wobei die Stegbreiten in unterschiedlichen Abschnitten des Stents dargestellt sind.

Der in den Figuren dargestellte Stent ist beispielsweise für den Einsatz der interventionellen Neuroradiologie geeignet, insbesondere zur Behandlung von Aneurysmen und Gefäßstenosen. Die Erfindung ist nicht auf Stents eingeschränkt, sondern umfasst allgemein medizinische Implantate, die in ein Körperhohlgefäß eingebracht werden. Die Erfindung ist beispielsweise auch auf Filter, Strömungsteiler oder andere medizinische Implantate anwendbar. Das Implantat, insbesondere der Stent kann selbstexpandierbar sein, und beispielsweise aus geeigneten Materialien, wie Nitinol oder anderen Formgedächtniswerkstoffen hergestellt sein. Der Stent kann auch ballonexpandierbar ausgebildet sein.

Das Implantat bzw. der Stent umfasst eine Wandung 10, die im implantierten Zustand mit der Gefäßwand in Berührung kommt und auf diese eine nach außen wirkende Radialkraft ausübt. Die Wandung 10 umfasst eine Gitterstruktur 12, die von einem komprimierten in einen expandierten Zustand überführbar ist. Dazu ist die Gitterstruktur in an sich bekannter Weise crimpbar und kann im komprimierten Zustand in einen Katheter geladen werden. Beim Implantieren entfaltet sich die Gitterstruktur 12 und ist in einen expandierten Zustand überführbar.

Die Gitterstruktur ist aus ersten Stegen 11 gebildet, die beispielsweise durch Laserschneiden, Ätzen oder andere Herstellungstechniken gefertigt sind. Die Stege 11 der Gitterstruktur 12 bilden geschlossene Zellen 13. Im Gegensatz zu offenen Zellen sind geschlossene Zellen mit den angrenzenden Nachbarzellen durch Verbinder 19 fest verbunden.

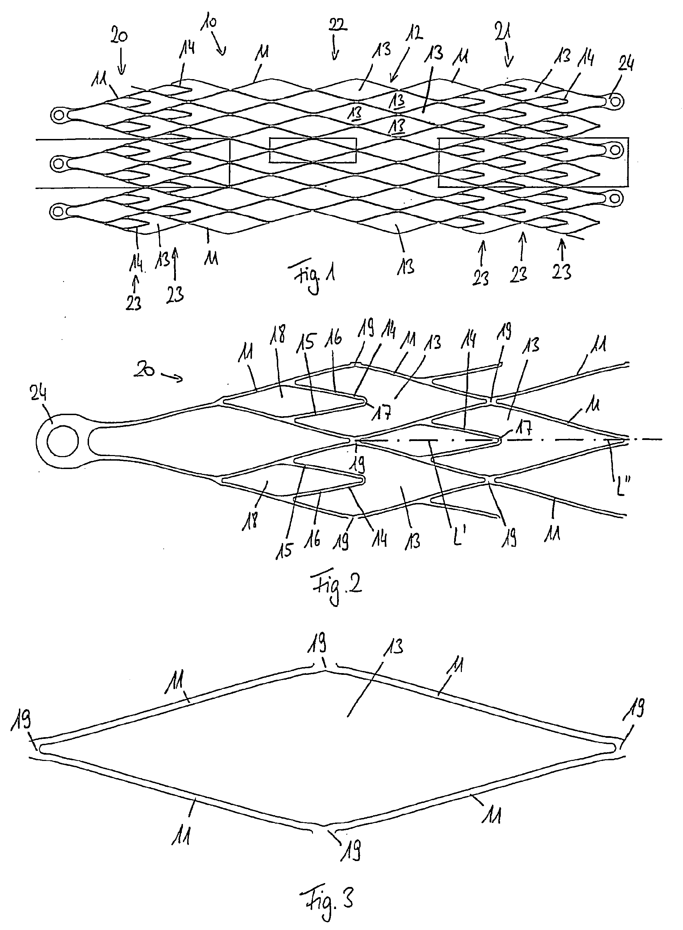

Wie in Fig. 2 gut zu erkennen, verbindet ein Verbinder 19 sowohl zwei in axialer Richtung angrenzende Zellen 13 als auch zwei in Umfangsrichtung aneinander angrenzende Zellen. Damit verbindet ein Verbinder 19 (bis auf die Randbereiche) vier Zellen 13.

Eine geschlossene Zelle bildet somit im Gegensatz zu einer offenen Zelle eine von Stegen 11 umgrenzte Zellöffnung, wobei die Stege 11 an allen Verbindungsstellen zu angrenzenden Zellen mit diesen fest verbunden sind. Dabei bilden die Verbinder 19 in Längsrichtung des Stents gesehen Verbindungsstellen, die axial nachgeordnete Zellen miteinander verbinden. In Umfangsrichtung gesehen bilden die Verbinder 19 ebenfalls Verbindungsstellen, die in Umfangsrichtung nachgeordnete Zellen miteinander fest verbinden.

Es ist auch möglich, dass die Gitterstruktur geschlossene und offene Zellen aufweist.

Ein Teil der geschlossenen Zellen 13 ist mit einem Halteelement 14 ausgebildet, das zur Verankerung der expandierten Gitterstruktur 12 in einem Gefäß angepasst ist. Es ist auch möglich, dass eine einzige Zelle 13 mehrere Halteelemente 14 aufweist. Die Halteelemente 14 sind jeweils aus wenigstens zwei zweiten Stegen 15, 16 gebildet, die einerseits miteinander verbunden sind und eine Spitze 17 aufweisen. Die Spitze 17 ragt in die Zelle 13 der Gitterstruktur hinein, wie in Fig. 2 zu erkennen. Die zweiten Stege 15, 16 sind andererseits mit den ersten Stegen 11 der Zelle 13 verbunden. Daraus ergibt sich ein stegförmiges Halteelement, das ein V-förmiges Profil aufweist, wobei die Spitze 17 des V-förmigen Profils in distale Richtung weist. Die beiden Stege 15, 16 divergieren von der Spitze 17 ausgehend und sind am Ende in etwa so breit, wie die Breite der die Zelle 13 aufspannenden Stege 11 im Bereich der Verbindungsstelle. Eine andere Anzahl an zweiten Stegen 15, 16 ist möglich. Die ersten Stege 11 der Gitterstruktur 12, die mit den zweiten Stegen 15, 16 des Halteelements 14 verbunden sind, weisen ebenfalls eine V-Form auf, die in entgegengesetzter Richtung zur V-Form des Halteelements 14 angeordnet ist.

Wie in Fig. 2 dargestellt, bilden die zweiten Stege 15, 16 des Halteelements 14 zusammen mit den ersten Stegen einer Zelle 13 der Gitterstuktur 12 eine weitere Zelle 18. Die kleinere Zelle 18 ist in der größeren Zelle 13 der Gitterstruktur 12 angeordnet, wobei die kleinere Zelle 18 teilweise durch die zweiten Stege 15, 16 des Halteelements und teilweise durch Stegabschnitte 11 der Gitterstruktur begrenzt wird. Wie in Fig. 2 gut zu erkennen, entspricht die Form der weiteren Zelle 18, d.h. der kleineren Zelle 18 der Form der Zelle 13 der Gitterstruktur 12, in der die weitere Zelle 18 angeordnet ist. Im Beispiel gemäß Fig. 2 handelt es sich in beiden Fällen um eine rautenförmige Zelle. Andere Zellgeometrien sind möglich. Die beiden zweiten Stege 15, 16 des Halteelements 14 greifen in etwa mittig an den ersten Stegen 11 der Zelle 13 an. Es ist auch möglich, die zweiten Stege 15, 16 des Halteelements 14 mit den ersten Stegen 11 der Zelle 13 an einer anderen, außermittigen Stelle zu verbinden, beispielsweise näher an der Spitze der Zelle 13 der Gitterstruktur 12 oder näher an den Verbindern 19. Das bedeutet, dass die zweiten Stege 15, 16 des Halteelements 14 jeweils an einer Position der zugeordneten ersten Stege 11 der Gitterstruktur 12 angreifen, die sich zwischen zwei Verbindern 19 befindet, die den jeweiligen ersten Steg 11 axial begrenzen. Der Begriff "axial" bezieht sich in diesem Zusammenhang auf die Längserstreckung des einzelnen Stegs. Das Halteelement 14 ist somit ein gesondertes Element, das zusätzlich zu den Verbindern 19 vorgesehen ist. Die Spitze 17 des Halteelements 14 ist zumindest auf der Höhe der seitlichen Verbindern 19 angeordnet. Im Ausführungsbeispiel gemäß Fig. 2 ragt die Spitze 17 über eine gedachte Verbindungslinie zwischen den beiden Verbindern 19 einer Zelle hinaus. Es ist auch möglich, die Spitze 17 hinter der gedachten Linie zwischen den beiden in Umfangsrichtung gegenüber angeordneten Verbindern 19 zu positionieren. Die Spitze 17 ist abgerundet, um möglichst atraumatisch die Verankerung des Implantats zu bewirken.

Bei dem Stentdesign gemäß Fig. 2 handelt es sich um ein achssymmetrisches Design, bezogen auf die einzelnen Zellen, bei denen die weiteren Zellen 18, die aus den Stegen 15, 16 der Halteelemente 14 gebildet sind, und die Zellen der Gitterstruktur 12 auf einer Linie liegen. Damit fluchtet die Längsachse L' der weiteren Zelle 18 mit der Längsachse L" der jeweils zugehörigen Zelle 13 der Gitterstruktur 12.

Die Spitzen der einzelnen Halteelemente 14 weisen jeweils in distale Richtung, wodurch das Wiedereinziehen des Stents in den Katheter erleichtert wird.

Wie in Fig. 1 dargestellt, weist ein Teil der geschlossenen Zellen 13 der Gitterstruktur 12 Halteelemente 14 auf. Es ist auch möglich, alle Zellen 13 mit Halteelementen 14 zu versehen. Bei dem Ausführungsbeispiel gemäß Fig. 1 sind zwei Randbereiche 20, 21 vorgesehen, bei denen die geschlossenen Zellen 13 Halteelemente 14 aufweisen. Der zwischen den beiden Randbereichen 20, 21 angeordnete Zwischenbereich 22 ist aus Zellen 13 ohne Halteelemente 14 gebildet. Damit sind die Halteelemente 14 so angeordnet, dass der Mittelbereich des Stents nicht an Porengröße einbüßt und die Durchlässigkeit der Zellen im Mittelbereich nicht beeinträchtigt wird. Die Verankerungsfunktion ist somit auf die Randbereiche beschränkt. Eine andere Anordnung der funktional verschiedenen Bereiche des Stents ist möglich. Konkret bilden die geschlossenen Zellen 13 mit den Halteelementen 14 Ringsegmente 23, die einander in axialer Richtung nachgeordnet sind. Die einzelnen Ringsegmente 23 setzen sich aus in Umfangsrichtung angeordneten geschlossenen Zellen zusammen, die jeweils durch seitliche Ver-binder 19 miteinander verbunden sind. Wie in Fig. 1 zu erkennen, ist die Anzahl der proximal angeordneten Ringsegmente 23 größer als die Anzahl der distal angeordneten Ringsegmente 23, wodurch eine präzise Positionierung des Stents beim Freisetzen verbunden mit einer vergleichsweise einfachen Wiedereinziehbarkeit erreicht wird. Bei dem Ausführungsbeispiel gemäß Fig. 1 sind auf der proximalen Stentseite 2 Ringsegmente 23 und auf der distalen Stentseite 3 Ringsegmente 23 vorgesehen. Eine andere Anzahl von Ringsegmenten 23 mit Halteelementen 14 ist sowohl proximal als auch distal möglich. Es ist auch möglich, dieselbe Anzahl von Ringsegmenten 23 mit Halteelementen 14 sowohl proximal als auch distal vorzusehen.

Die beiden Randbereiche 20, 21 sind jeweils mit Markern, insbesondere mit Röntgenmarkern 24 verbunden.

Die geschlossenen Zellen 13 der Gitterstruktur 12 weisen eine rautenförmige oder diamantartige Geometrie auf, wobei die einzelnen Schenkel der Rautengeometrie durch die Stege 11 der Gitterstruktur gebildet sind. Der Formwinkel der einzelnen Zelle 13, d.h. der Winkel zwischen der Längsachse der Zelle und der Verbindungslinie zwischen einem seitlichen und einem die Spitze der Zelle bildenden Verbinder 19, beträgt vorzugsweise 50°. Der Formwinkel kann ≥ 25°, ≥ 30°, ≥ 35°, ≥ 40° betragen. Die Obergrenze des Formwinkels kann ≤ 60°, ≤ 55°, ≤ 50°, ≤ 45° betragen. Die vorstehend angegebenen Werte des Formwinkels beziehen sich auf den Ruhezustand.

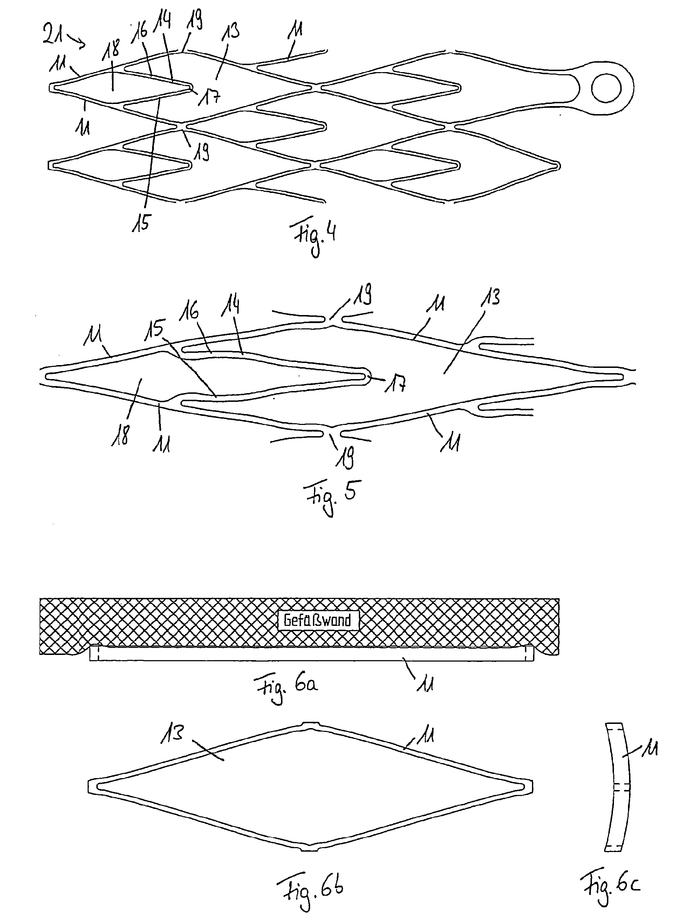

In Fig. 5 ist eine teilverformte Zelle 13 mit Halteelement 14, das entsprechend der Zelle 13 verformt wird, dargestellt.

Die Funktionsweise der Erfindung wird nachfolgend anhand der Figuren 6 bis 8 erläutert.

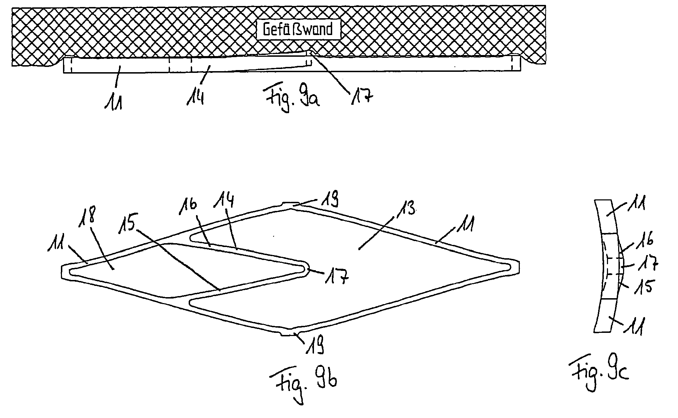

Wie in Fig. 6a, 6b und 6c dargestellt, führt ein reines Closed Cell Design, d.h. eine geschlossene Zelle 13 ohne Halteelement 14 dazu, dass die Gefäßwand relativ stark gespannt wird mit der Folge, dass sich die Stege 11 der geschlossenen Zelle 13 schwächer in die Gefäßwand eindrücken. Im Unterschied dazu prägt sich, wie in Fig. 9a dargestellt, das Halteelement 14 stärker in die Gefäßwand ein, das im implantierten Zustand über die Wandung des Stents bzw. allgemein des Implantats teilweise radial nach außen vorsteht. Durch die abgerundete atraumatische Spitze 17 werden dabei Verletzungen der Gefäßwand vermieden. Die nach außen gerichtete radiale Auslenkung des Halteelements 14 im expandierten Zustand ist auch in Fig. 9c dargestellt. Der vorstehend genannte Effekt kann durch geeignete Dimensionierung bzw. geometrische Gestaltung des Halteelements 14 verstärkt werden, wie in den Figuren 9a, 9b und 9c verdeutlicht. Darin ist zu erkennen, dass sich das Halteelement 14 auch bei rein axialer Ausrichtung des Stents über die Wandung 10 erhebt, so dass die atraumatisch geformte Spitze 17 weiter in die Gefäßwand eingedrückt wird als die Verbinder 19.

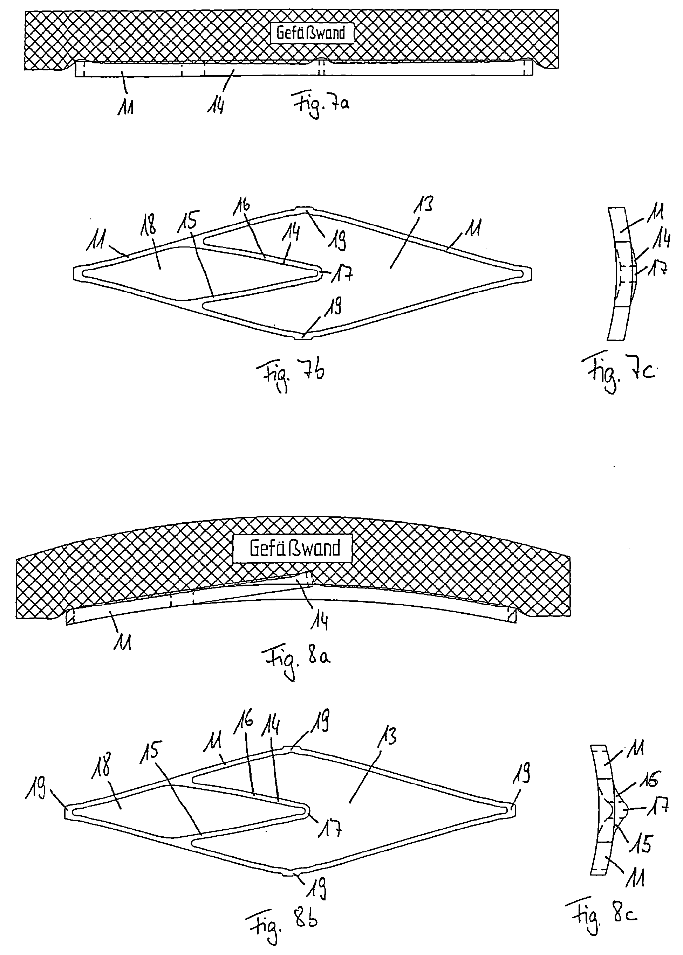

Es ist auch möglich, das Halteelement 14 derart auszubilden, dass die Spitze 17 im expandierten Zustand nicht über die Wandung 10 hinausragt, sondern in der Ebene zwischen den Stegen 11 verbleibt, wie in Fig. 7a, 7b und 7c dargestellt. Die radiale Auslenkung des Halteelements 14 wird durch das Verhältnis der Stegbreite zur Wandstärke der Stege 11 eingestellt. Dadurch ist es möglich, die Lage der Spitze 17 im expandierten Zustand derart zu beeinflussen, dass diese entweder in der Wandung 10 verbleibt oder nach außen über die Wandung 10 vorsteht.

Im Fall, dass das Halteelement 14 nicht radial ausgelenkt wird und die Spitze 17 in der Wandungsebene verbleibt, hat das Halteelement 14 eine Haltefunktion. Die Haltefunktion des Halteelements 14 ergibt sich in diesem Fall daraus, dass die Stege 11 und das Halteelement 14 sich in die Intima der Gefäßwand pressen, wodurch sich ein entsprechender Widerstandseffekt ergibt.

Die Verankerung des Stents mittels der Halteelemente 14 ist besonders ausgeprägt, wenn der Stent in Krümmung geht, wie in Fig. 8a, 8b, 8c dargestellt. Dabei werden die Halteelemente 14 noch stärker aus der Wandungsebene herausbewegt und prägen sich tiefer in die Gefäßwand ein. Dadurch wird das Risiko einer Dislokation des Stents in distaler Richtung noch weiter verringert.

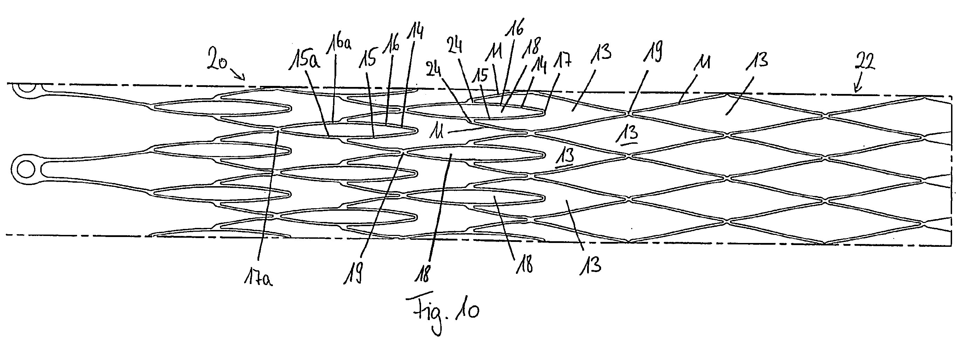

Ein weiteres Ausführungsbeispiel des erfindungsgemäßen Stents ist in der Draufsicht gemäß Fig. 10 dargestellt. Darin ist ein Umfangssegment des Stents im aufgeklappten Zustand gezeigt, wobei das dargestellte Umfangssegment einem Teil des Zwischenbereichs 22 sowie einem Umfangsabschnitt des Randbereichs 20 umfasst. Der Zwischenbereich 22 ist aus geschlossenen Zellen 13 gebildet, die mit jeweils vier Verbindern 19 mit benachbarten Zellen 13 sowohl in axialer, als auch in Umfangsrichtung gekoppelt sind. Die geschlossenen Zellen 13 sind im Wesentlichen rautenförmig ausgebildet.

Im Unterschied zu den halteelementfreien geschlossenen Zellen 13 des Zwischenbereichs 22 sind im Randbereich 20 geschlossene Zellen 13 angeordnet, die mit Halteelementen 14 ausgestattet sind. Die Halteelemente 14 sind dabei zwischen zwei ersten Stegen 11 der Gitterstruktur 12 aufgespannt. Insbesondere sind die Halteelemente 14 bzw. die zweiten Stege 15, 16 der Halteelemente 14 jeweils mit axialen Enden 24 der ersten Stege 11 gekoppelt. Dabei bildet das axiale Ende 24 eines ersten Stegs 11 die Begrenzung des Stegs 11 in axialer Richtung bezogen auf die Längserstreckung des einzelnen Stegs 11 und geht unmittelbar in den Verbinder 19 über. Analog zu den zuvor beschrieben Ausführungsbeispielen bildet das Halteelement 14, insbesondere die zweiten Stege 15, 16 mit der Spitze 17, ein V-förmiges Profil, das sich in die geschlossene Zelle 13 hineinerstreckt.

Bei dem Ausführungsbeispiel gemäß Fig. 10 sind die ersten Stege 11, die mit dem Halteelement 14 gekoppelt sind, im Unterschied zu den freien ersten Stegen 11, die miteinander direkt mit Verbindern 19 verbunden sind, verkürzt. Im Allgemeinen weisen also die mit dem Halteelement 14 gekoppelten ersten Stege 11 eine andere axiale Länge auf als die direkt bzw. unmittelbar über Verbinder 19 gekoppelten ersten Stege 11. Es ist auch möglich, dass die mit dem Halteelement 14 verbundenen ersten Stege 11 dieselbe Länge aufweisen wie die freien ersten Stege 11.

Wie in Fig. 10 weiter zu erkennen, sind die ersten Stege 11 im Wesentlichen an den zweiten Stegen 15, 16 angelenkt, wobei das Halteelement 14 sich derart zwischen den ersten Stegen 11 erstreckt, dass das Halteelement 14 im Wesentlichen einen Verbinder 19 der Zelle 13 ersetzt. Zusätzlich sind die zweiten Stege 15, 16 in Längsrichtung des Stents verlängert und bilden jeweils eine Stegverlängerung 15a, 16a. Die Stegverlängerungen 15a, 16a konvergieren und sind zu einer gemeinsamen weiteren Spitze 17a zusammengeführt. Insgesamt ist das Halteelement 14 also symmetrisch ausgebildet, wobei die Symmetrieachse durch die axialen Enden 24 der ersten Stege 11 festgelegt ist. Die Form des Halteelements 14 lässt sich als blattfederartig oder linsenförmig beschreiben. Die zweiten Stege 15, 16 bilden also mit der jeweiligen Stegverlängerung 15a, 16a jeweils ein Kreisbogensegment, wobei die Kreisbogensegmente an den beiden Längsenden bezogen auf die Stentlängsachse miteinander gekoppelt sind und die Spitze 17 bzw. die weitere Spitze 17a bilden.

Im Vergleich zu den zuvor beschriebenen Ausführungsbeispielen, insbesondere gemäß Fig. 2 und Fig. 5, ist also das Halteelement 14 in Richtung des näher gelegenen Stentendes relativ zur geschlossenen Zelle 13 verschoben, so dass der Verbindungspunkt zwischen den ersten Stegen 11 und den zweiten Stegen 15, 16 mit der Lage des Verbinders 19 zusammenfällt, der die geschlossene Zelle 13 in Längsrichtung des Stents begrenzt.

In einer alternativen Sichtweise auf das Ausführungsbeispiel gemäß Fig. 10 kann die geschlossene Zelle 13 bzw. die Grundzelle als durch die ersten Stege 11 und die Stegverlängerungen 15a, 16a gebildet angesehen werden. In dieser Sichtweise ist das Halteelement 14 lediglich aus den zweiten Stegen 15, 16 gebildet, die zu einer Spitze 17 zusammengeführt sind. Der Unterschied zu den übrigen, oben aufgeführten Ausführungsbeispielen besteht dann darin, dass die ersten Stege 11 mit der Stegverlängerung 15a, 16a nicht fluchtend angeordnet sind. Vielmehr sind die Stegverlängerungen 15a, 16a, die in dieser Sichtweise als Teil der ersten Stege 11 angesehen werden, zu den zweiten Stegen 15, 16 fluchtend ausgerichtet. Somit bilden die ersten Stege 11 an der Verbindungsstelle zum Halteelement 14 jeweils einen Knick bzw. eine Krümmung, die den Übergang zwischen den ersten Stegen 11 und der Stegverlängerung 15a, 16a bildet.

Unabhängig von der Sichtweise, wie das Ausführungsbeispiel gemäß Fig. 10 zu beschreiben ist, bilden die zweiten Stege 15, 16 mit den Stegverlängerungen 15a, 16a, insbesondere einschließlich der Spitze 17 und der weiteren Spitze 17a, eine weitere, innere Zelle 18, die zumindest teilweise in der Grundzelle bzw. geschlossenen Zelle 13 angeordnet ist.

Wie in Fig. 10 weiter zu erkennen, unterscheidet sich die Form der ersten Stege 11 im Zwischenbereich von der Form der ersten Stege 11 im Randbereich. Im Zwischenbereich sind die ersten Stege 11 im Wesentlichen geradlinig ausgebildet. Das bedeutet, dass die Verbinder 19 im Zwischenbereich 22 durch im Wesentlich geradlinig bzw. gerade verlaufende erste Stege 11 gekoppelt sind. Die ersten Stege 11 erstrecken sich also zwischen den Verbindern 19 im Zwischenbereich 22 zumindest in der Umfangsebene des Stents ungekrümmt. Die ersten Stege 11 im Randbereich weisen hingegen in der Nähe der Verbinder 19, die die Zelle 13 in Längsrichtung begrenzen, eine Krümmung auf und/oder bilden im Bereich des Verbinders 19 eine Verdickung. Insbesondere sind die ersten Stege 11 zueinander V-förmig ausgerichtet, wobei die V-förmige Spitze im Bereich des Verbinders 19 endet und der Winkel zwischen den ersten Stegen 11 variiert, insbesondere mit größerem Abstand zum Verbinder 19 aufgeweitet wird. Es hat sich allgemein gezeigt, dass insbesondere mit der Struktur des Stents gemäß dem Ausführungsbeispiel nach Fig. 10 eine stress- bzw. spannungsoptimierte Funktion erreichbar ist.

Insgesamt beruhen die in den Figuren dargestellten Ausführungsbeispiele auf einer im Wesentlichen rautenförmigen Zellgeometrie, in der speziell ausgeformte, insbesondere stegförmige Halteelemente 14 derart in den Randbereichen des Stents angeordnet sind, dass die Verankerung des Stents verbessert und ein Verrutschen des Stents während der Intervention vermieden wird. Durch die Anordnung der Halteelemente 14 in den Randbereichen des Stents wird ferner erreicht, dass im Mittelbereich des Stents, d.h. im Bereich zwischen den Randbereichen die Porengröße beibehalten wird, so dass die Durchlässigkeit der Zellen in diesem Bereich nicht beeinträchtigt wird. Die Anordnung der Spitzen 17 der Halteelemente in distaler Richtung führt dazu, dass ein Widerhakeneffekt bewirkt wird, wobei gleichzeitig das Wiedereinziehen der Halteelemente 14 in einen Katheter des bis zu 80% entlassenen Stents möglich ist. Dies ist bei Stents, die auf der Grundlage eines Open Cell Designs hergestellt sind, nicht möglich.

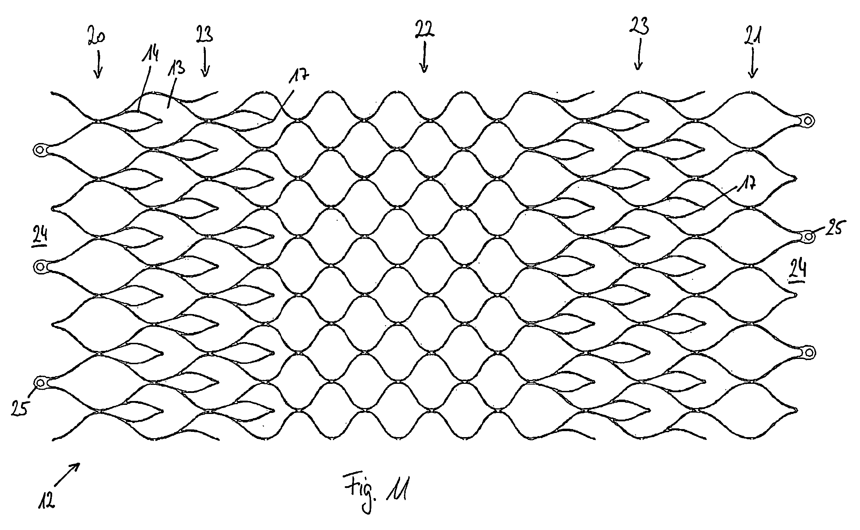

Fig. 11 zeigt einen Stent nach einem erfindungsgemäßen Ausführungsbeispiel im abgewickelten Zustand. Der Stent gemäß Fig. 11 entspricht im Wesentlichen dem Stent gemäß Fig. 1, wobei jedoch die ersten Stege 11 einen S-förmigen Verlauf aufweisen.

Der Stent gemäß Fig. 11 umfasst zwei Randbereiche 20, 21, die mit Röntgenmarkern 25 versehen sind. Die Randbereiche 20, 21 bilden jeweils die axialen Enden 24 des Stents. Insbesondere ist an den axialen Enden 24 des Stents jeweils eine Reihe in Umfangsrichtung benachbart angeordneter geschlossener Zellen 13 vorgesehen, wobei jede zweite geschlossene Zelle einen Röntgenmarker 25 trägt. An die Randbereiche 20, 21 schließen sich jeweils Ringsegmente 23 an. Bei dem Ausführungsbeispiel gemäß Fig. 11 sind drei Reihen in Umfangsrichtung benachbarter geschlossener Zellen 13 vorgesehen, die jeweils ein Ringsegment 23 bilden. Die Ringsegmente 23 unterscheiden sich von den übrigen Segmenten des Stents dadurch, dass die geschlossenen Zellen 13 in den Ringsegmenten 23 jeweils ein Halteelement 14 umfassen. Zwischen den Ringsegmenten 23 erstreckt sich ein Zwischenbereich 22, der ein oder mehrere Reihen in Umfangsrichtung benachbarter geschlossener Zellen 13 umfasst. Die geschlossenen Zellen 13 des Zwischenbereichs 22 sind halteelementfrei ausgebildet. Mit anderen Worten weisen die geschlossenen Zellen 13 des Zwischenbereichs 22 keine Halteelemente 14 auf.

Wie in Fig. 11 ferner zu erkennen ist, weisen die Spitzen 17 der Halteelemente 14 in den Ringsegmenten 23 jeweils in dieselbe Richtung. Besonders bevorzugt ist es, wenn die Spitzen 17 aller Halteelemente 14 der Ringsegmente 23 bzw. allgemein des Stents in dieselbe Richtung, insbesondere in distale Richtung bezogen auf ein Zuführsystem weisen. In Fig. 11 weist die distale Richtung entsprechend der Zeichnung nach rechts, wogegen die proximale Richtung nach links weist. Mit anderen Worten bilden die geschlossenen Zellen 13 des Randbereichs 21 im rechten Zeichnungsabschnitt das distale Ende des Stents, wogegen die geschlossenen Zellen 13 des Randbereichs 20 in der linken Zeichnungshälfte das proximale Ende des Stents bilden.

Vorzugsweise sind die geschlossenen Zellen 13 der Randbereiche 20, 21, der Ringsegmente 23 sowie des Zwischenbereichs 22 derart ausgebildet, dass der Stent über die gesamte Länge eine konstante, insbesondere gleichförmige, Radialkraft aufweist. Konkret sind insbesondere die Form und/oder die Größe der einzelnen geschlossenen Zellen 13 in den jeweiligen Segmenten bzw. Bereichen der Gitterstruktur 12 derart ausgelegt, dass die normierte Radialkraft, also die Radialkraft pro Stentlänge, bzw. der Radialdruck über die gesamte Länge bzw. den Umfang des Stents im Wesentlichen konstant bleibt. Beispielsweise sind die geschlossenen Zellen 13, die Halteelemente 14 umfassen, größer als geschlossene Zellen 13 ausgebildet, die keine Halteelemente 14 umfassen, um die durch die Halteelemente 14 erhöhte Radialkraft auszugleichen.

Die geschlossenen Zellen 13 mit Halteelementen 14 in den Randbereiche 20,21 der Gitterstruktur 12 können gegenüber den geschlossenen Zellen 13 eines mittleren Bereichs, insbesondere der Ringsegmente 23, der Gitterstruktur 12, die keine Haltelemente 14 aufweisen, eine andere Radialkraft umfassen. Die Radialkraft kann dabei durch die Geometrie der Zelle 13 beeinflusst werden. Beispielsweise kann die Länge der Zelle 13 - entlang der Längsachse der Gitterstruktur betrachtet - oder der Winkel der ersten Stege 11 einer Zelle 13 variiert werden, um die Radialkräfte in unterschiedlichen Abschnitten der Gitterstruktur 12 einzustellen. Die Radialkraft in den Randbereichen 20, 21 kann gegenüber der Radialkraft in einem mittleren Bereich der Gitterstruktur 12 kleiner oder größer sein. Es ist auch möglich, dass die Zellen 13 in den Randbereichen 20, 21 derart geometrisch dimensioniert sind, dass sie die gleiche Radialkraft wie die Zellen 13 eines mittleren Bereichs der Gitterstruktur 12 aufweisen. Insbesondere kann die Radialkraft entlang der Gitterstruktur 12 konstant sein.

Die Einstellung der Radialkräfte entlang der Gitterstruktur 12 ist abhängig von der Anwendung der medizinischen Vorrichtung und wird vom Fachmann entsprechend gewählt. Dabei kann berücksichtigt werden, dass eine Radialkraft in den Randbereichen 20, 21 der Gitterstruktur 12, wobei die Zellen 12 in den Randbereichen 20, 21 Halteelemente 14 aufweisen, das Nebenwirkungsrisiko, beispielsweise die Gefahr einer Stenosenbildung, im Randbereich 20, 21 der implantierten medizinischen Vorrichtung reduziert.

Im Allgemeinen wird durch die Halteelemente 14 die Verformungskraft der jeweils zugeordneten Zelle 13 erhöht. Bei einer radialen Komprimierung des Stents bzw. der Gitterstruktur 12 gegenüber dem Ruhezustand verformen sich nicht nur die ersten Stege 11 der Zelle, sondern auch die zweiten Stege 15, 16 des Halteelements. Gegenüber einer Zelle, die halteelementfrei ausgebildet ist, also kein Halteelement 14 umfasst, erhöht sich die Radialkraft durch die Anwesenheit eines Halteelements 14, selbst wenn die Zelle 13 dieselben geometrischen Dimensionen aufweist.

Es ist möglich, dass die Zellen 13 in einem Randbereich 20, 21 der Gitterstruktur 12 Verankerungszellen 13a, also Zellen 13 Halteelementen 14, umfassen, die das gleiche Profil und die gleichen Dimensionsverhältnisse (Stegbreite, Steglänge, Zellenwinkel, Zellenbreite,...) wie die halteelementfreien Zellen 13, also die Freizellen 13b, in einem mittleren Bereich (ohne Halteelement) aufweisen. In diesem Fall ist die Radialkraft im Randbereich 20, 21 durch die zusätzlichen Halteelemente 14 größer als im mittleren Bereich.

Es kann vorteilhaft sein, wenn die Radialkraft im Randbereich 20, 21 der Gitterstruktur 12 reduziert bzw. an die Radialkraft im mittleren Bereich der Gitterstruktur 12 angeglichen ist. Dadurch wird ein sanfter Übergang bezogen auf die mechanischen Eigenschaften der Zellen 13 zwischen dem mittleren Bereich (Freizellen 13b) und den Randbereichen (Verankerungszellen 13a) erzielt. Insbesondere können die mechanischen Eigenschaften, insbesondere die Radialkraft, der Randbereiche 20, 21 und des mittleren Bereichs identisch sein. Ein abrupter, durch sehr unterschiedliche Kräfte gekennzeichneter Übergang kann zu einer hohen lokalen Beanspruchung des Gefäßes im Übergangsbereich führen, da sich die Pulsatilitätswelle bzw. Verformungswelle des Gefäßes, die durch den pulsierenden Blutfluss bewirkt wird, auf beiden Seiten des Übergangsbereichs stark ändern kann.

In einer bevorzugten Ausführungsform weisen die Zellen 13 im Randbereich 20, 21 eine andere bzw. unterschiedliche geometrische Form als die Zellen 13 im mittleren Bereich auf. Das bewirkt, dass eine im Randbereich 20, 21 angeordnete Zelle 13 an sich, also ohne Berücksichtigung des Halteelements, eine kleinere Radialkraft als eine Zelle 13 im mittleren Bereich der Gitterstruktur 12 bewirkt. Das wird u.a. durch einen kleineren Tip-Winkel, also dem Winkel zwischen zwei ersten Stegen im Bereich eines Verbinders, erzielt und/oder durch eine kleinere Stegbreite und/oder durch längere Stege und/oder durch eine kleineren Anzahl von Zellen 13 in einer Umfangsreihe und/oder durch unterschiedliche Steggeometrien, insbesondere unterschiedliche Krümmungsradien der ersten Stege. Dabei kann der Winkel der ersten Stege 11 der Zellen 13 in einem mittleren Bereich der Gitterstruktur 12 um vorzugsweise höchstens 15°, insbesondere höchstens 10°, insbesondere höchstens 5°, kleiner als der Winkel der ersten Stege 11 der Zellen 13 in einem Randbereich 20, 21 der Gitterstruktur 12 sein. Dadurch wird dem Effekt der Radialkrafterhöhung durch die Halteelemente 14 entgegengewirkt.

Insgesamt sind folgende, unterschiedliche Möglichkeiten vorgesehen:
Es ist möglich, dass die Radialkraft in einem Randbereich 20, 21 der Gitterstruktur 12 (Zellen 13 mit Halteelementen) größer als in einem mittleren Bereich der Gitterstruktur 12 ist. Durch eine entlang der Gitterstruktur 12 bzw. des Stents, insbesondere im Randbereich, sich ändernde Geometrie der Zellen 13 die Radialkraft schrittweise und oder graduell ansteigt. Ein abrupter Übergang im Zwischenbereich 22 wird dadurch vermieden.

Es ist ferner möglich, dass die Radialkraft in einem Randbereich 20, 21 (Zellen 13 mit Halteelemente) durch die Änderung der Geometrie an die mechanischen Eigenschaften, insbesondere die Radialkraft, in einem mittleren Bereich angeglichen bzw. auf den gleichen Wert eingestellt ist. Beispielsweise kann nur eine oder mehrere erste Umfangsreihen bzw. ein oder mehrere erste Umfangssegmente der Gitterstruktur 12, die Verankerungszellen 13a umfassen und mit Freizellen 13b eines mittleren Bereiches verbunden sind, die gleiche Radialkraft wie die Umfangsreihen bzw. Umfangssegmente eines mittleren Bereichs der Gitterstruktur 12 aufweisen. In weiteren Randsegmenten bzw. Randbereichen, die von den Verankerungszellen 13a des mittleren Bereiches getrennt bzw. beabstandet angeordnet sind, kann die Radialkraft graduell ansteigen.

Außerdem besteht die Möglichkeit, dass in einem Randbereich 20, 21 angeordnete Umfangsreihen bzw. Umfangssegmente, die Zellen 13 mit Halteelementen (Verankerungszellen 13a) aufweisen, oder zumindest einige dieser Umfangssegmente mit Verankerungszellen 13a eine geringere Radialkraft als Umgangssegmente in einem mittleren Bereich der Gitterstruktur 12 aufweisen, die keine Halteelemente 14 umfassen bzw. durch Freizellen 13b gebildet sind. Dadurch wird die Radialkraft in dem Randbereich 20, 21 der Gitterstruktur 12 bzw. des Stents reduziert. Insbesondere kann die Radialkraft in dem Randbereich 20, 21 der Gitterstruktur 12 schrittweise und/oder graduell in Richtung des axialen Endes der Gitterstruktur 12 gesenkt werden, um im implantierten Zustand die Gefäßwände zumindest in den Abschnitten zu schonen, in denen der Randbereich 20, 21 der Gitterstruktur 12 angeordnet ist.

Eine Kombination von Zellen 13 und Umfangsegmenten bzw. Umfangsreihen der Randbereiche 20, 21 mit niedrigerer, gleicher oder höherer Radialkraft gegenüber den Zellen 13 eines mittleren Bereichs der Gitterstruktur 12 ist möglich.

Die Reduzierung der Radialkraft in den Umfangssegmenten, die Zellen 13 mit Halteelementen umfassen, ist hinsichtlich der Anhaftung des Stents bzw. der Gitterstruktur 12 an einer Gefäßwand ist möglich, da die Halteelemente 14 eine "geometrischen Arretierung bzw. Anhaftung ermöglichen. Das bedeutet, dass die Arretierung zumindest hauptsächlich durch die geometrische Form der Halteelemente 14 bewirkt wird. Die Form der Halteelemente 14 ermöglicht eine gute Anhaftung auch bei vergleichsweise niedriger Radialkraft der zugeordneten Zellen.

Zur Messung der Radialkraft können übliche Radialkraftmesssysteme eingesetzt werden, die mehrere Lamellen aufweisen, die sich blendenartig nach innen schließen.
Es ist auch möglich, separate Zellen 13 zu untersuchen. Die separaten Zellen 13 können an einer Zugmaschine gedehnt bzw. gedrückt werden. Aus dem Wert der Verformungskraft lässt sich der Wert der Radialkraft des Stents hochrechnen.

Insgesamt weist die Gitterstruktur 12 Reihen S_{HE} geschlossener Zellen 13 mit Halteelementen 14 sowie Reihen S geschlossener Zellen 13 ohne Halteelemente 14 auf. Vorzugsweise sind in den axialen Endabschnitten des Stents bzw. der Gitterstruktur 12 jeweils zwei, drei, vier, fünf, sechs, sieben, acht oder neun Reihen S_{HE} geschlossener Zellen 13 mit Halteelementen 14 vorgesehen. Insgesamt kann das Verhältnis zwischen Reihen S_{HE} geschlossener Zellen 13 mit Halteelementen 14 zu Reihen S geschlossener Zellen 13 ohne Halteelemente 14 (S_{HE}/S) einen Bereich von 2/4 bis 2/10, insbesondere 3/3 bis 3/12, insbesondere 4/4 bis 4/10, insbesondere 5/5 bis 5/15, insbesondere 6/4 bis 6/16, insbesondere 7/5 bis 7/24, insbesondere 8/4 bis 8/20, insbesondere 9/5 bis 9/27, insbesondere 10/4 bis 10/30, insbesondere 12/4 bis 12/32, umfassen.

Die geschlossenen Zellen 13, die ein Halteelement 14 umfassen, werden nachfolgend als Verankerungszellen 13a bezeichnet. Geschlossene Zellen 13, die keine Halteelemente 14 umfassen, werden Freizellen 13b genannt. Ferner werden Übergangszellen 13c unterschieden, die jeweils zwischen einer Reihe von Verankerungszellen 13a und einer Reihe von Freizellen 13b angeordnet sind und den Übergang zwischen den unterschiedlichen Dimensionen der Verankerungszellen 13a und den Freizellen 13b bewirken. Die Übergangszellen 13c können Halteelemente 14 aufweisen.

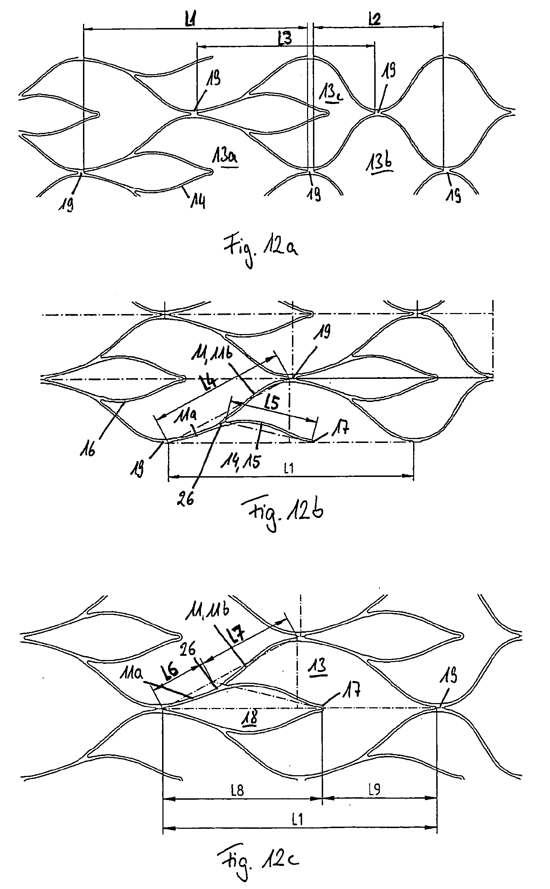

In Fig. 12a sind unterschiedliche Längen bzw. Abstände innerhalb der Gitterstruktur 12 dargestellt. Dabei bezeichnen die Bezugszeichen L1 bis L8 folgende Längen bzw. Abstände:
Wie in Fig. 12a dargestellt, entspricht eine Verankerungszellenlänge L1 dem Abstand zweier Verbinder 19 einer Verankerungszelle 13a. Dabei verbinden die Verbinder 19 jeweils wenigstens zwei erste Stege 11 der Verankerungszelle 13a. Die Freizellen 13b weisen eine Freizellenlänge L2 auf, die dem Abstand zweier Verbinder 19 der Freizelle 13b entspricht. Vorzugsweise ist die Freizellenlänge L2 kleiner als die Verankerungszellenlänge L1 ausgebildet. Die Übergangszelle 13c umfasst ferner eine Übergangszellenlänge L3, die ebenfalls dem Abstand zweier Verbinder 19 der Übergangszelle 13c entspricht. Die Übergangszellenlänge L3 ist vorzugsweise größer als die Freizellenlänge L2 und kleiner als die Verankerungszellenlänge L1. Die Verankerungszellenlänge L1, die Freizellenlänge L2 und die Übergangszellenlänge L3 beziehen sich jeweils auf den Abstand zweier Verbinder 19, die in Längsrichtung der Gitterstruktur 12 benachbart, insbesondere fluchtend, angeordnet sind.

Gemäß Fig. 12b ist ferner ein Verbinderabstand L4 ermittelbar, der dem Abstand zweier Verbinder 19 entspricht, die durch einen gemeinsamen ersten Steg 11 gekoppelt sind. Der Verbinderabstand L4 erstreckt sich somit im Wesentlichen schräg zur Längsachse bzw. zur Längsrichtung der Gitterstruktur 12. Aus Figur 12b ist überdies ein Spitzenabstand L5 ableitbar, der dem Abstand der Spitze 17 des Halteelements 14 zu einem Ansatzpunkt 26 entspricht. Die Spitze 17 und der Ansatzpunkt 26 bilden jeweils ein Ende des zweiten Stegs 15, 16 des Halteelements 14. Der Ansatzpunkt 26 entspricht also der Stelle, an dem der zweite Steg 15, 16 des Halteelements 14 dem ersten Steg 11 der geschlossenen Zelle 13 entspringt.

Ferner ist gemäß Fig. 12c ein Ansatzabstand L6 vorgesehen, der im Wesentlichen dem Abstand zwischen einem Ansatzpunkt 26 und einem Verbinder 19 entspricht, wobei der Verbinder 19 der weiteren Zelle 18 zugeordnet ist, der auch der Ansatzpunkt 26 angehört. Die weitere Zelle 18 wird durch zwei Stegansatzabschnitte 11a zweier erster Stege 11, die in dem Verbinder 19 gekoppelt sind, und die zweiten Stege 15, 16 des Halteelements 14 gebildet. Mit anderen Worten bildet das Halteelement 14 gemeinsam mit jeweils einem Stegansatzabschnitt 11a zweier erster Stege 11 die weitere Zelle 18, die in die geschlossene Zelle 13 hineinragt. Der Ansatzpunkt 26 weist zu einem weiteren Verbinder 19, der mit dem Ansatzpunkt 26 durch einen Reststegabschnitt 11b verbunden ist, einen Reststegabstand L7 auf. Der Reststegabstand L7 entspricht also im Wesentlichen dem Abstand zwischen dem Ansatzpunkt 26 und einem Verbinder 19, wobei der Abstand entlang des Reststegabschnitts 11b gemessen wird. Insgesamt sind die ersten Stege 11, aus denen die zweiten Stege 15, 16 des Halteelements 14 entspringen, in zwei Abschnitte, nämlich den Stegansatzabschnitt 11a und den Reststegabschnitt 11b, unterteilt. Der Stegansatzabschnitt 11a ist Teil der weiteren Zellen 18, die durch das Halteelement 14 gebildet wird. Der Reststegabschnitt 11b ist hingegen der geschlossenen Zelle 13 zugeordnet.

Der Abstand zwischen der Spitze 17 des Haltelementes 14 und einem Verbinder 19, der der weiteren Zelle 18, die durch das Halteelement 14 gebildet ist, zugeordnet ist, entspricht der Halteelementlänge L8, wie in Fig. 12c dargestellt. Mit anderen Worten wird die Länge der weiteren Zellen 18 in axialer Richtung bzw. in Längsrichtung der Gitterstruktur 12 als Halteelementlänge L8 bezeichnet. Die Spitze 17 des Halteelements 14 fluchtet zusätzlich mit einem weiteren Verbinder 19 der geschlossenen Zelle 13. Der Abstand zwischen der Spitze 17 und einem weiteren Verbinder 19, der in Längsrichtung der Gitterstruktur 12 mit der Spitze 17 fluchtet und nicht dem Halteelement 14 zugeordnet ist, dem die Spitze 17 zugeordnet ist, wird als Öffnungslänge L9 bezeichnet. Die Öffnungslänge L9 entspricht der Differenz zwischen der Verankerungszellenlänge L1 und der Halteelementlänge L8.

In diesem Zusammenhang wird darauf hingewiesen, dass sich die zuvor genannten Längen bzw. Abstände L4-L9 mit Ausnahme Verankerungszellenlänge L1, der Freizellenlänge L2 und der Übergangszellenlänge L3 grundsätzlich auf die Dimensionen aller geschlossener Zellen 13 beziehen können. Die Längen- bzw. Abstandsangaben sind also nicht auf eine besondere Form der Zelle, also nicht auf eine Verankerungszelle 13, eine Freizelle 13b oder eine Übergangszelle 13c, beschränkt. Den Abständen L4-L7 ist grundsätzlich eine geradlinige Verbindung, in den Figuren als strichpunktierte Linie dargestellt, zugrunde gelegt. Überdies wird darauf hingewiesen, dass sich alle Längen und Abstandsangaben auf den vollständig expandierten Zustand der Gitterstruktur 12, also den Herstellzustand, beziehen.

Folgende Längen bzw. Abstandsverhältnisse sind bevorzugt:
Das Verhältnis zwischen der Verankerungszellenlänge L1 und der Freizellenlänge L2 (L1/L2) beträgt vorzugsweise wenigstens 0,7, insbesondere wenigstens 0,8, insbesondere wenigstens 0,9, insbesondere wenigstens 1. Vorzugsweise beträgt das Verhältnis zwischen Verankerungszellenlänge L1 und Freizellenlänge L2 (L1/L2) höchstens 2,5, insbesondere höchstens 2,4, insbesondere höchstens 2,3, insbesondere höchstens 2,2, insbesondere höchstens 2,1, insbesondere höchstens 2.

Das Verhältnis zwischen der Übergangszellenlänge L3 und der Freizellenlänge L2 (L3/L2) beträgt vorzugsweise wenigstens 0,6, insbesondere wenigstens 0,7, insbesondere wenigstens 0,8, insbesondere wenigstens 0,9. Als Obergrenze für das Verhältnis zwischen der Übergangszellenlänge L3 und der Freizellenlänge L2 (L3/L2) ist vorzugsweise ein Wert von höchstens 2,5, insbesondere höchstens 2,3, insbesondere höchstens 2,1, insbesondere höchstens 2, insbesondere höchstens 1,8, insbesondere höchstens 1,6, insbesondere höchstens 1,5 vorgesehen.

Für das Verhältnis zwischen der Verankerungszellenlänge L1 und dem Verbinderabstand L4 (L1/L4) ist vorzugsweise ein Wert von mindestens 1, insbesondere mindestens 1,2, insbesondere mindestens 1,4, insbesondere mindestens 1,5, vorgesehen. Vorzugsweise beträgt das Verhältnis zwischen Verankerungszellenlänge L1 und Verbinderabstand L4 (L1/L4) höchstens 3, insbesondere höchstens 2,8, insbesondere höchstens 2,6, insbesondere höchstens 2,4.

Die Übergangszellenlänge L3 kann zum Verbinderabstand L4 ein Verhältnis (L3/L4) aufweisen, das wenigstens 0,9, insbesondere wenigstens 1, insbesondere wenigstens 1,1, insbesondere wenigstens 1,2, beträgt. Vorzugsweise umfasst das Verhältnis zwischen der Übergangszellenlänge L3 und dem Verbinderabstand L4 (L3/L4) höchstens 2,5, insbesondere höchstens 2,4, insbesondere höchstens 2,3, insbesondere höchstens 2,2, insbesondere höchstens 2,1, insbesondere höchstens 2,0.

Eine Untergrenze für das Verhältnis zwischen dem Verbinderabstand L4 und dem Spitzenabstand L5 (L4/L5) beträgt vorzugsweise wenigstens 0,8, insbesondere wenigstens 0,9, insbesondere wenigstens 1, insbesondere wenigstens 1,0. Der Maximalwert für das Verhältnis zwischen dem Verbinderabstand L4 und dem Spitzenabstand L5 (L4/L5) ist vorzugsweise auf höchstens 2,5, insbesondere höchstens 2,4, insbesondere höchstens 2,3, insbesondere höchstens 2,2, insbesondere höchstens 2,1, insbesondere höchstens 2, festgelegt.

Das Verhältnis zwischen dem Ansatzabstand L6 und dem Reststegabstand L7 (L6/L7) beträgt vorzugsweise wenigstens 0,2, insbesondere wenigstens 0,3, insbesondere wenigstens 0,4, insbesondere wenigstens 0,5. Für das Verhältnis zwischen Ansatzabstand L6 und Reststegabstand L7 (L6/L7) ist vorzugsweise ein Maximalwert von höchstens 2, insbesondere höchstens 1,8, insbesondere höchstens 1,6, insbesondere höchstens 1,4, insbesondere höchstens 1,2, insbesondere höchstens 1, insbesondere höchstens 0,8, insbesondere höchstens 0,6, vorgesehen.

Die Haltelementlänge L8 verhält sich zur Verankerungszellenlänge L1 vorzugsweise in einem Verhältnis (L8/L1) von wenigstens 0,2. Das Verhältnis zwischen Halteelementlänge L8 und Verankerungszellenlänge L1 (L8/L1) beträgt vorzugsweise höchstens 1,0, insbesondere höchstens 0,75, insbesondere höchstens 0,7, insbesondere höchstens 0,65, insbesondere höchstens 0,6. Die vorgenannten Höchstwerte gelten vorzugsweise auch für das Verhältnis zwischen der Öffnungslänge L9 und der Verankerungszellenlänge L1. Konkret beträgt das Verhältnis zwischen der Öffnungslänge L9 und der Verankerungszellenlänge L1 (L9/L1) vorzugsweise höchstens 1,0, insbesondere höchstens 0,75, insbesondere höchstens 0,7, insbesondere höchstens 0,65, insbesondere höchstens 0,6. Die Untergrenze für das Verhältnis zwischen der Öffnungslänge L9 und der Verankerungszellenlänge L1 (L9/L1) beträgt vorzugsweise wenigstens 0,2.

Die Halteelementlänge L8 und die Öffnungslänge L9 stehen vorzugsweise in einem Verhältnis (L8/L9) zueinander, das wenigstens 0,6, insbesondere wenigstens 0,7, insbesondere wenigstens 0,8, insbesondere wenigstens 0,9, insbesondere wenigstens 1, beträgt. Die Obergrenze für das Verhältnis zwischen Haltelementlänge L8 und Öffnungslänge L9 (L8/L9) beträgt vorzugsweise höchstens 2, 5, insbesondere höchstens 2,2, insbesondere höchstens 2,0, insbesondere höchstens 1,8, insbesondere höchstens 1,6, insbesondere höchstens 1,5.

Der Verbinderabstand L4, der Spitzenabstand L5, der Ansatzabstand L6, der Reststegabstand L7, die Halteelementlänge L8 und die Öffnungslänge L9 können sich jeweils auf unterschiedliche Ausgestaltungen der geschlossenen Zellen 13, insbesondere sowohl auf die Verankerungszelle 13a, als auch die Freizelle 13b, als auch die Übergangszelle 13c, beziehen.

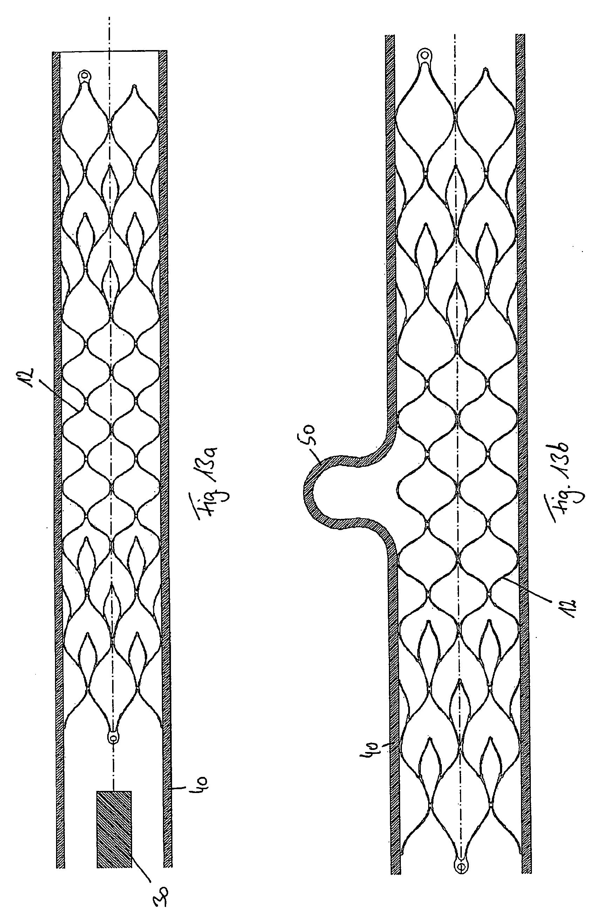

Fig. 13a zeigt den Stent gemäß Fig. 11 im implantierten Zustand innerhalb eines Körperhohlorgans 40. Der Stent bzw. die Gitterstruktur 12 ist in dem Körperhohlorgan 40 expandiert und liegt an den Gefäßwänden des Körperhohlorgans 40 an. Ferner ist in Fig. 13 die Spitze eines Zuführsystems 30 dargestellt, mit dem der Stent in das Körperhohlorgan 40 eingeführt wurde. In Fig. 13a ist deutlich zu erkennen, dass alle Halteelemente 14 der Gitterstruktur 12 bzw. deren Spitzen 17 in distale Richtung, also vom Zuführsystem 30 weg, weisen. Dadurch ist ermöglicht, den Stent bzw. die Gitterstruktur 12 in das Zuführsystem 30 zurückzuführen, insbesondere wenn die Gitterstruktur 12 nur teilweise aus dem Zuführsystem 30 entlassen ist. Durch die in gleicher Richtung, insbesondere in distale Richtung orientierten Halteelemente 14 wird ein Verhaken der Halteelemente 14 beim Zurückziehen in das Zuführsystem 30 verhindert.

Die Verankerung der medizinischen Vorrichtung bzw. der Gitterstruktur 12 in einem Körperhohlorgan 40, insbesondere einem Blutgefäß, erfolgt vorwiegend durch die geometrischen Verhältnisse in den Zellen 13, die Halteelemente 14 aufweisen. Durch geeignete Auslegung der ersten und zweiten Stege 11, 15, der Länge der Zellen 13 sowie der Wandstärke und der Stegbreiten wird die verbesserte Verankerung im Blutgefäß bzw. allgemein Körperhohlraum 40 erreicht. Insbesondere ist durch geeignete geometrische Auslegung der Grad der radialen Auslenkung der Halteelemente 14 beeinflussbar. Die Haltelemente 14 können derart konstruiert sein, dass eine Auslenkung nur durch eine Krümmung der Gitterstruktur 12 entlang einer Längsachse erfolgt.

Ferner ermöglicht die Ausrichtung der Halteelemente 14, insbesondere aller Halteelemente 14 in distale Richtung eine verbesserte Verankerung der Gitterstruktur 12 bzw. des Implantats im Körperhohlraum 40. Dieser Vorteil kommt insbesondere bei Verwendung des Stents bzw. der Gitterstruktur 12 als Coilstent zum Tragen.

Coilstents dienen zur Abdeckung eines Aneurysmas 50, wie in Fig. 13b dargestellt. Im Allgemeinen hat es sich als vorteilhaft erwiesen, durch die Zellen 13 der Gitterstruktur 12 einen Katheter in das Aneurysma 50 zu führen, um Coils in das Aneurysma 50 einzubringen. Dabei besteht die Gefahr, dass der Coilkatheter in die Gitterstruktur 12 einhakt und die Gitterstruktur 12 durch eine axiale Bewegung des Coilkatheters im Körperhohlorgan 40 verschoben wird. Durch die Halteelemente 14 wird jedoch eine zusätzliche Verankerung der Gitterstruktur 12 in der Gefäßwand des Körperhohlorgans 40 erreicht, so dass das Risiko einer Verschiebung bzw. Dislokation der Gitterstruktur 12 gesenkt bzw. minimiert wird. Die Gitterstruktur 12 gemäß Fig. 11, die in Verwendung als Coilkatheter in Fig. 13b dargestellt ist, eignet sich zur Abdeckung von Aneurysmen 50 besonders, da die Halteelemente 14 im Bereich der axialen Endabschnitte der Gitterstruktur 12 angeordnet sind, die außerhalb des Aneurysmas 50, vorzugsweise in den gesunden Bereichen des Körperhohlorgans 40, positionierbar sind.

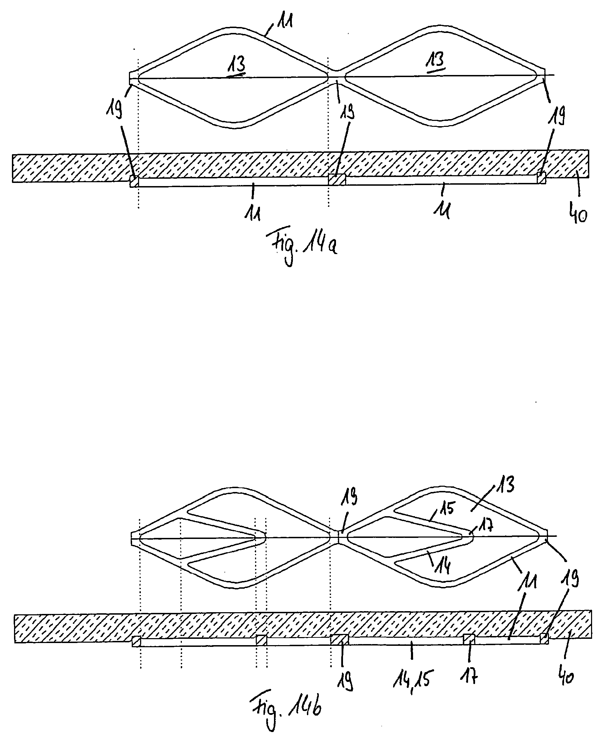

Im Allgemeinen bewirkt die Radialkraft der Gitterstruktur 12, dass sich die Stege 11 der Gitterstruktur 12 in die Gefäßwand des Körperhohlorgans 40 zumindest teilweise eindrücken. Durch die Radialkraft, die von der Gitterstruktur 12 ausgehend auf das Körperhohlorgan 40 wirkt, ergibt sich eine Art Hinterschnitt, wie in Fig. 14a dargestellt. Mit anderen Worten werden die Stege 11 der Gitterstruktur 12 zumindest teilweise von der Gefäßwand des Körperhohlorgans 40 umschlossen. Auf diese Weise wird eine Verankerung der Gitterstruktur 12 in der Gefäßwand des Körperhohlorgans 40 bewirkt. Insbesondere wirkt der vorgenannte Hinterschnitt gegen eine axiale Dislokation des Stents. Dieser Effekt wird bei geschlossenen Zellen 13, die ein Halteelement 14 umfassen, verstärkt, wie in Fig. 14b erkennbar ist. Zusätzlich zu den ersten Stegen 11 werden im implantierten Zustand auch die Halteelemente 14 zumindest teilweise in die Gefäßwand des Körperhohlorgans 40 eingedrückt, wodurch sich die gesamte, in die Gefäßwand hineindrückende Oberfläche der Gitterstruktur 12 erhöht. Dies führt zu einer verbesserten Verankerung und einer höheren Widerstandskraft gegen eine axiale Dislokation.

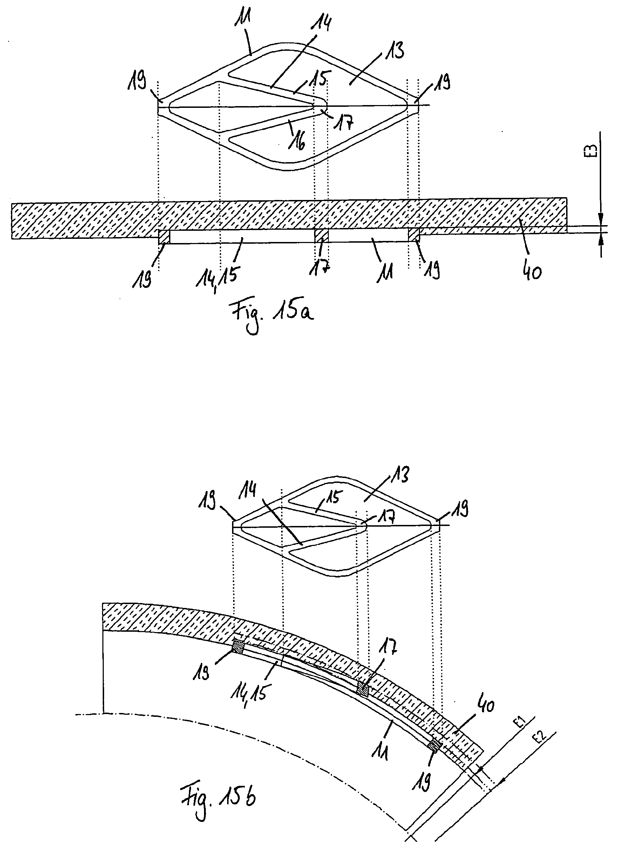

Eine weitere Verstärkung des vorgenannten Effekts tritt bei Anordnung der Gitterstruktur 12 in gekrümmten Gefäßabschnitten auf. In Fig. 15a ist die Verankerung einer geschlossenen Zelle 13 mit einem Halteelement 14 in einer Gefäßwand eines Körperhohlorgans 40 dargestellt. Dabei ergibt sich eine Eindrücktiefe E3, die den Betrag wiedergibt, um den die geschlossene Zelle 13 in die Gefäßwand einsinkt bzw. eingedrückt wird. Bei einer Anordnung der geschlossenen Zelle 13 gemäß Fig. 15a in einer Gefäßkrümmung, wie in Fig. 15b dargestellt, ergibt sich eine Eindringtiefe E2 des Halteelements 14, die vom Ansatzpunkt 26 ausgehend bis zur Spitze 17 hin zunimmt und größer als die Eindringtiefe E1 der ersten Stege 11 ist. Das Halteelement 14 ist also in Anordnung in einer Gefäßkrümmung gegenüber den ersten Stegen 11 bzw. der geschlossenen Zelle 13 radial nach außen ausgelenkt. Somit wird die Verankerung der Gitterstruktur 12 in einer Gefäßkrümmung verbessert.

Vorzugsweise weisen die ersten Stege 11 der geschlossenen Zelle 13 und die zweiten Stege 15, 16 des Halteelements 14 unterschiedliche Stegbreiten auf. Insbesondere ist vorzugsweise ein Verhältnis zwischen der Stegbreite S1 der ersten Stege 11 und der Stegbreite S2 der zweiten Stege 15, 16 (S1/S2) vorgesehen, das wenigstens 0,5 und höchstens 2 beträgt. Dabei umfasst die Stegbreite S1 der ersten Stege 11 und/oder die Stegbreite S2 der zweiten Stege 15, 16 vorzugweise einen Wert von wenigstens 0,010 mm, insbesondere wenigstens 0,015 mm, insbesondere wenigstens 0,020 mm, insbesondere wenigstens 0,025 mm. Die Stegbreite S1 der ersten Stege 11 und/oder die Stegbreite S2 der zweiten Stege 15, 16 kann höchstens 0,06 mm, insbesondere höchstens 0,08 mm, insbesondere höchstens 0,07 mm, insbesondere höchstens 0,06 mm, betragen.

Die Wandung 10 des Implantats weist vorzugsweise eine Wandstärke W auf, die wenigstens 0,03 mm, insbesondere wenigstens 0,04 mm, insbesondere wenigstens 0,05 mm und/oder höchstens 0,09 mm, insbesondere höchstens 0,08 mm, insbesondere höchstens 0,07 mm, insbesondere höchstens 0,06 mm, insbesondere höchstens 0,055 mm, beträgt. Vorteilhafterweise ist ein Verhältnis zwischen der Wandstärke W und der Stegbreite S1 der ersten Stege 11 oder der Stegbreite S2 der zweiten Stege 15, 16 (W/S1 bzw. W/S2) vorgesehen, das wenigstens 0,8, insbesondere wenigstens 0,9, insbesondere wenigstens 1, insbesondere wenigstens 1,1, insbesondere wenigstens 1,2, insbesondere wenigstens 1,3, und/oder höchstens 2,0, insbesondere höchstens 1,8, insbesondere höchstens 1,6, insbesondere höchstens 1,4, beträgt.

Der Querschnittsdurchmesser des Implantats bzw. der Gitterstruktur 12 beträgt im expandierten Zustand bzw. Herstellzustand vorzugsweise wenigstens 1,5 mm, insbesondere wenigstens 1,75 mm, insbesondere wenigstens 2,0 mm, insbesondere wenigstens 2,25 mm, insbesondere wenigstens 2,5 mm. Als Obergrenze für den expandierten Durchmesser D des Implantats bzw. der Gitterstruktur 12 ist ein Wert von höchstens 6,5 mm, insbesondere höchstens 5,5 mm, insbesondere höchstens 5,0 mm, insbesondere höchstens 4,5 mm, insbesondere höchstens 4,0 mm, insbesondere höchstens 3,5 mm, insbesondere höchstens 3,0 mm vorgesehen.

Ferner wird ein bevorzugter Einsatzdurchmesser angegeben, der dem bevorzugten Durchmesser im implantierten Zustand entspricht und vorzugsweise wenigstens 1,0 mm, insbesondere wenigstens 1,5 mm, insbesondere wenigstens 2,0 mm beträgt. Der bevorzugte Einsatzdurchmesser kann höchstens 6,0 mm, insbesondere höchstens 5,5 mm, insbesondere höchstens 4,5 mm, insbesondere höchstens 4,0 mm, insbesondere höchstens 3,5 mm, insbesondere höchstens 2,5 mm, betragen. Die Stentlänge beträgt vorzugsweise wenigstens 10 mm, insbesondere wenigstens 12 mm, insbesondere wenigstens 14 mm, insbesondere wenigstens 15 mm. Eine Maximallänge des Stents bzw. eine maximale Stentlänge beträgt vorzugsweise höchstens 150 mm, insbesondere höchstens 140 mm, insbesondere höchstens 130 mm, insbesondere höchstens 120 mm, insbesondere höchstens 100 mm, insbesondere höchstens 80 mm, insbesondere höchstens 60 mm, insbesondere höchstens 40 mm, insbesondere höchstens 20 mm.

### Bezugszeichenliste

- 10: Wandung
- 11: erste Stege
- 11a: Stegansatzabschnitt
- 11b: Reststegabschnitt
- 12: Gitterstruktur
- 13: geschlossene Zelle
- 13a: Verankerungszelle
- 13b: Freizelle
- 13c: Übergangszelle
- 14: Halteelement
- 15a, 16a: Stegverlängerung
- 15, 16: zweite Stege
- 17: Spitze
- 17a: weitere Spitze
- 18: weitere Zelle
- 19: Verbinder
- 20, 21: Randbereiche
- 22: Zwischenbereich
- 23: Ringsegmente
- 24: axiales Ende
- 25: Röntgenmarker
- 26: Ansatzpunkt
- 30: Zuführsystem
- 40: Körperhohlorgan
- 50: Aneurysma

- L': Längsachse
- L": Längsachse

- L1: Verankerungszellenlänge
- L2: Freizellenlänge
- L3: Übergangszellenlänge
- L4: Verbinderabstand
- L5: Spitzenabstand
- L6: Ansatzabstand
- L7: Reststegabstand
- L8: Halteelementlänge
- L9: Öffnungslänge

- E1: Eindringtiefe der ersten Stege 11
- E2: Eindringtiefe des Halteelements 14
- E3: Eindrücktiefe
- S1: Stegbreite der ersten Stege 11

- S2: Stegbreite der zweiten Stege 15, 16
- W: Wandstärke

## Patentansprüche

1. Medizinisches Implantat mit einer Wandung (10), die eine von einem komprimierten in einen expandierten Zustand überführbare und aus ersten Stegen (11) gebildete Gitterstruktur (12) umfasst, wobei die Gitterstruktur (12) geschlossene Zellen (13) mit jeweils einem Halteelement (14) aufweist, das zur Verankerung der expandierten Gitterstruktur (12) in einem Gefäß angepasst ist und wenigstens zwei zweite Stege (15, 16) aufweist, die einerseits miteinander verbunden sind und eine Spitze (17) bilden, die in die Zelle (13) ragt, und andererseits mit den ersten Stegen (11) der Zelle (13) verbunden sind, und wobei die ersten Stege (11) der Gitterstruktur (12) mit den zweiten Stegen (15,16) des Halteelements (14) jeweils zwischen zwei die ersten Stege (11) der Gitterstruktur (12) axial begrenzenden Verbindern (19) verbunden sind.

2. Implantat nach Anspruch 1,
**dadurch gekennzeichnet, dass**
die zweiten Stege (15, 16) des Halteelements (14) mit den ersten Stegen einer Zelle (13) der Gitterstruktur (12) eine weitere Zelle (18) bilden, die in der Zelle (13) der Gitterstruktur (12) angeordnet ist.

3. Implantat nach Anspruch 2,
**dadurch gekennzeichnet, dass**
die Form der weiteren Zelle (18) der Form der Zelle (13) der Gitterstruktur (12) entspricht.

4. Implantat nach wenigstens einem der Ansprüche 1 bis 3,
**dadurch gekennzeichnet, dass**
eine Längsachse L' der weiteren Zelle (18) mit einer Längsachse L" der zugehörigen Zelle (13) der Gitterstruktur (12) fluchtet.

5. Implantat nach wenigstens einem der Ansprüche 1 bis 4,
**dadurch gekennzeichnet, dass**
die Spitze (17) des Halteelements (14) zumindest auf der Höhe seitlicher Ver-binder (19) angeordnet ist, die in Umfangsrichtung nebeneinander angeordnete Zellen (13) der Gitterstruktur (12) verbinden.

6. Implantat nach wenigstens einem der Ansprüche 1 bis 5,
**dadurch gekennzeichnet, dass**
die Spitze (17) in distale Richtung des Implantats weist.

7. Implantat nach wenigstens einem der Ansprüche 1 bis 6,
**dadurch gekennzeichnet, dass**
in Längsrichtung der Wandung (10) wenigstens ein Randbereich (20), insbesondere zwei Randbereiche (20, 21), und wenigstens ein Zwischenbereich (22) der Gitterstruktur (12) in Längsrichtung der Wandung (10) angeordnet sind, wobei die Halteelemente (14) im Randbereich (20, 21) ausgebildet sind.

8. Implantat nach wenigstens einem der Ansprüche 1 bis 7,
**dadurch gekennzeichnet, dass**
die geschlossenen Zellen (13) der Gitterstruktur (12) mehrere in axialer Richtung der Wandung (10) einander nachgeordnete Ringsegmente (23) bilden, wobei die Zellen (13) eines Ringsegments (23) wenigstens ein Halteelement (14), insbesondere alle Zellen (13) des Ringsegments (23) jeweils ein Halteelement (14), aufweisen.

9. Implantat nach Anspruch 8,
**dadurch gekennzeichnet, dass**
die Anzahl der distal angeordneten Ringsegmente (23) mit Halteelementen (14) größer ist als die Anzahl der proximal angeordneten Ringsegmente (23) mit Halteelementen (14).

10. Implantat nach wenigstens einem der Ansprüche 1 bis 9,
**dadurch gekennzeichnet, dass**
die Zelle (13) der Gitterstruktur (12) eine rautenförmige Zelle umfasst.

11. Implantat nach wenigstens einem der Ansprüche 1 bis 10,
**dadurch gekennzeichnet, dass** die weitere Zelle (18), die in der Zelle (13) der Gitterstruktur (12) angeordnet ist, eine rautenförmige Zelle umfasst.

12. Implantat nach wenigstens einem der Ansprüche 1 bis 11,
**dadurch gekennzeichnet, dass**
das Halteelement (14) angepasst ist derart, dass sich die Spitze (17) des Halteelements (14) bei einer Krümmung der Gitterstruktur (12) entlang einer Längsachse der Gitterstruktur (12) selbsttätig radial nach außen aufstellt.

13. Implantat nach wenigstens einem der Ansprüche 1 bis 12,
**dadurch gekennzeichnet, dass**
mehrere Halteelemente (14) vorgesehen sind, die über den Umfang der Gitterstruktur (12) verteilt angeordnet sind, wobei sich bei einer Krümmung der Gitterstruktur (12) entlang einer Längsachse der Gitterstruktur (12) die Spitzen (17) der Haltelemente (14), insbesondere nur der Halteelemente (14), radial nach außen aufstellen, die auf einer vom Krümmungsmittelpunkt abgewandten Seite der Gitterstruktur (12) angeordnet sind.

14. Implantat nach wenigstens einem der Ansprüche 1 bis 13,
**dadurch gekennzeichnet, dass**
mehrere Halteelement (14) vorgesehen sind, wobei die Spitzen (17) aller Halteelemente (14) in dieselbe Richtung, insbesondere in distale Richtung bezogen auf ein Zuführsystem (30) weisen.

## Claims

1. Medical implant with a wall (10) which comprises a lattice structure (12) which is convertable from a compressed into an expanded state and is formed of first webs (11), wherein the lattice structure (12) comprises closed cells (13) each with a holding element (14) which is adapted for anchoring the expanded lattice structure (12) in a vessel, and at least two other webs (15, 16), which on the one hand are connected to each other and form a tip (17) which projects into the cells (13), and on the other hand are connected with the first webs (11) of the cell (13), and wherein the first webs (11) of the lattice structure (12) are connected to the second webs (15, 16) of the holding element (14) in each case between two connectors (19) axially delimiting the first webs (11) of the lattice structure (12).

2. Implant according to claim 1,
**characterised in that**
the second webs (15, 16) of the holding element (14) together with the first webs of a cell (13) of the lattice structure (12) form a further cell (18) which is arranged in the cell (13) of the lattice structure (12).

3. Implant according to claim 2,
**characterised in that**
the shape of the further cell (18) corresponds to the shape of the cell (13) of the lattice structure (12).

4. Implant according to at least one of claims 1 to 3,
**characterised in that**
the longitudinal axis L' of the further cell (18) is in alignment with a longitudinal axis L" of the associated cell (13) of the lattice structure (12).

5. Implant according to at least one of claims 1 to 4,
**characterised in that**
the tip (17) of the supporting element (14) is at least arranged at lateral connectors (19) which connect in the circumferential direction adjacently arranged cells (13) of the lattice structure (12).

6. Implant according to at least one of claims 1 to 5,
**characterised in that**
the tip (17) points in the distal direction of the implant.

7. Implant according to at least one of claims 1 to 6,
**characterised in that**
in the longitudinal direction of the wall (10) at least one marginal area (20), more particularly two marginal areas (20, 21), and at least one intermediate area (22) of the lattice structure (12) are arranged in the longitudinal direction of the wall (10), wherein the holding elements (14) are provided in the marginal area (20, 21).

8. Implant according to at least one of claims 1 to 7,
**characterised in that**
the closed cells (13) of the lattice structure (12) form several ring segments (23) arranged one after the other in the axial direction of the wall (10), wherein the cells (13) of one ring segment (23) have at least one holding element (14), more particularly all cells (13) of the ring segment (23) each have a holding element (14).

9. Implant according to claim 8,
**characterised in that**
the number of distally arranged ring segments (23) with holding elements (14) is greater than the number of proximally arranged ring segments (23) with holding elements (14).

10. Implant according to at least one of claims 1 to 9,
**characterised in that**
the cell (13) of the lattice structure (12) comprises a rhombus-shaped cell.

11. Implant according to at least one of claims 1 to 10,
**characterised in that**
the further cell (18), which is arranged in the cell (13) of the lattice structure (12), comprises a rhombus-shaped cell.

12. Implant according to at least one of claims 1 to 11,
**characterised in that** the
holding element (14) is adapted in such a way that on bending the lattice structure (12) along a longitudinal axis of the lattice structure (12) the tip (17) of the holding element (14) automatically erects outwards in a radial direction.

13. Implant according to any one of claims 1 to 12,
**characterised in that**
several holding elements (14) are provided, which are distributed around the circumference of the lattice structure (12), wherein on bending of the lattice structure (12) along a longitudinal axis of the lattice structure (12) the tips (17) of the holding elements (14), more particularly only of the holding elements (14) which are arranged on a side of the lattice structure (12) facing away from the centre of curvature, erect outwards in a radial direction.

14. Implant according to at least one of claims 1 to 13,
**characterised in that**
several holding elements (14) are provided, wherein the tips (17) of all the holding elements (14) point in the same direction, more particularly in the distal direction with regard to an insertion system (30).

## Revendications

1. Implant médical comportant une paroi (10) qui comprend une structure grillagée (12) pouvant passer d'un état comprimé à un état déployé et formée de premières traverses (11), la structure grillagée (12) comportant des cellules fermées (13) dotées chacune d'un élément de retenue (14) qui est apte à ancrer la structure grillagée déployée (12) dans un vaisseau, et au moins deux secondes traverses (15, 16) qui sont d'une part reliées entre elles et forment une pointe (17) qui dépasse dans la cellule (13) et sont reliées d'autre part aux premières traverses (11) de la cellule (13), les premières traverses (11) de la structure grillagée (12) étant reliées aux secondes traverses (15, 16) de l'élément de retenue (14) respectivement entre deux connecteurs (19) limitant axialement les premières traverses (11) de la structure grillagée (12).

2. Implant selon la revendication 1,
**caractérisé en ce que**
les secondes traverses (15, 16) de l'élément de retenue (14) forment avec les premières traverses d'une cellule (13) de la structure grillagée (12) une autre cellule (18) qui est disposée dans la cellule (13) de la structure grillagée (12).

3. Implant selon la revendication 2,
**caractérisé en ce que**
la forme de l'autre cellule (18) correspond à la forme de la cellule (13) de la structure grillagée (12).

4. Implant selon une des revendications 1 à 3,
**caractérisé en ce**
**qu'**un axe longitudinal L' de l'autre cellule (18) est aligné avec un axe longitudinal L" de la cellule correspondante (13) de la structure grillagée (12).

5. Implant selon au moins une des revendications 1 à 4,
**caractérisé en ce que**
la pointe (17) de l'élément de retenue (14) est disposée au moins à la hauteur de connecteurs latéraux (19) qui relient entre elles des cellules juxtaposées dans le sens circonférentiel (13) de la structure grillagée (12).

6. Implant selon au moins une des revendications 1 à 5,
**caractérisé en ce que**
la pointe (17) est orientée dans le sens distal de l'implant.

7. Implant selon au moins une des revendications 1 à 6,
**caractérisé en ce que**
dans le sens longitudinal de la paroi (10), au moins une zone périphérique (20), en particulier deux zones périphériques (20, 21), et au moins une zone intermédiaire (22) de la structure grillagée (12) sont disposées dans le sens longitudinal de la paroi (10), les éléments de retenue (14) étant réalisés dans la zone périphérique (20, 21).

8. Implant selon au moins une des revendications 1 à 7,
**caractérisé en ce que**
les cellules fermées (13) de la structure grillagée (12) constituent plusieurs segments annulaires (23) disposés les uns derrière les autres dans le sens axial de la paroi (10), chacune des cellules (13) d'un segment annulaire (23) présentant au moins un élément de retenue (14), en particulier chacune de toutes les cellules (13) de l'élément annulaire (23) présentant un élément de retenue (14).

9. Implant selon la revendication 8,
**caractérisé en ce que**
le nombre de segments annulaires disposés de manière distale (23) avec des éléments de retenue (14) est supérieur au nombre de segments annulaires disposés de manière proximale (23) avec des éléments de retenue (14).

10. Implant selon une des revendications 1 à 9,
**caractérisé en ce que**
la cellule (13) de la structure grillagée (12) comprend une cellule en forme de losange.

11. Implant selon au moins une des revendications 1 à 10,
**caractérisé en ce que**
l'autre cellule (18) qui est disposée dans la cellule (13) de la structure grillagée (12) comprend une cellule en forme de losange.

12. Implant selon au moins une des revendications 1 à 11,
**caractérisé en ce que**
l'élément de retenue (14) est adapté de manière à ce que la pointe (17) de l'élément de retenue (14), en cas de courbure de la structure grillagée (12) le long d'un axe longitudinal de la structure grillagée (12), se pose automatiquement radialement vers l'extérieur.

13. Implant selon au moins une des revendications 1 à 12,
**caractérisé en ce**
**qu'**il est prévu plusieurs éléments de retenue (14) qui sont disposés répartis sur la circonférence de la structure grillagée (12), sachant que, en cas de courbure de la structure grillagée (12) le long d'un axe longitudinal de la structure grillagée (12), les pointes (17) des éléments de retenue (14), en particulier seulement des éléments de retenue (14) qui sont disposés sur une face détournée d'un point central de courbure de la structure grillagée (12), se posent radialement vers l'extérieur.

14. Implant selon au moins une des revendications 1 à 13,
**caractérisé en ce**
**qu'**il est prévu plusieurs éléments de retenue (14), les pointes (17) de tous les éléments de retenue (14) étant orientées dans le même sens, en particulier dans le sens distal relativement un système d'acheminement (30).
